# EUROPEAN PATENT SPECIFICATION

(11) **EP 3 545 924 B1**
(45) Date of publication and mention of the grant of the patent: **07.09.2022**
(21) Application number: 19164092.9
(22) Date of filing: 20.03.2019
(51) Int. Cl.: A61F 13/496, B29C 65/08, A61F 13/15, B29C 65/56

(54) **ABSORBENT ARTICLE**
ABSORBIERENDER ARTIKEL
ARTICLE ABSORBANT

(30) Priority: 28.03.2018 JP 2018062468
(43) Date of publication of application: 02.10.2019
(73) Proprietor: Unicharm Corporation, Shikokuchuo-shi, Ehime 799-0111 (JP)
(72) Inventor: KAWABATA, Kuniyoshi, Kagawa, Kagawa 769-1602 (JP); ICHIKAWA, Makoto, Kagawa, Kagawa 769-1602 (JP); MUKAI, Hirotomo, Kagawa, Kagawa 769-1602 (JP)
(74) Representative: Staeger & Sperling Partnerschaftsgesellschaft mbB

(56) References cited:
- EP-A1- 2 412 354
- WO-A1-2008/041203
- WO-A1-2018/225302

## Description

### FIELD

The present invention relates to an absorbent article.

### BACKGROUND

An absorbent article (for example, a pants-type disposable diaper) is known that includes an abdomen side section and a dorsal side section, with both edges of the abdomen side section and dorsal side section in the transverse direction overlapping in the thickness direction along their longitudinal directions, and being joined together at a pair of joint sections. A method of forming the pair of joint sections of the absorbent article is disclosed in Patent Literature 1 (Japanese Unexamined Patent Publication No. 2004-298413; U.S. Patent Publication No. US2005145317(A1)), for example, as a method of forming joint sections (seal sections) using a sealing apparatus. In this method, while the sheet members are transported, prescribed locations of the sheet members where the joint sections are to be formed are clamped and fused between an ultrasonic horn and anvil during the short period when the prescribed locations pass through the gap between the ultrasonic horn and anvil, thereby the joint sections are formed at the prescribed locations. In this case, a plurality of fused locations, i.e. fused sections, are formed at the joint sections, aligned along the longitudinal direction.

### [CITATION LIST]

### [PATENT LITERATURE]

[PTL 1] Japanese Unexamined Patent Publication No. 2004-298413. Further prior art in this technical field is disclosed in documents EP 2 412 354 A1, WO 2008/041203 A1 and WO 2018/225302 A1.

### SUMMARY

### [TECHNICAL PROBLEM]

When the used absorbent article is removed from the wearer, the usual method employed is to peel apart the abdomen side section and the dorsal side section along the longitudinal direction at each pair of joint sections. That is, at each joint section, the abdomen side section and dorsal side section are separated from each other at each of the plurality of fused sections that are aligned along the longitudinal direction.

According to research by the present inventors, adjusting the various sorts of conditions in the method of Patent Literature 1 allows formation of each of fused sections at the joint section so that it has a core section where the sheet members are pressed while being fused, and a perimeter wall section where the sheet members are fused, surrounding the core section in a tubular manner. Perimeter wall sections are preferably provided at the fused sections to help stably prevent separation of the joint sections by movement of the wearer while the absorbent article is being worn. Furthermore, when the fused sections have perimeter wall sections, and the absorbent article is removed, i.e. the abdomen side section and dorsal side section are peeled apart from each other, the fused sections remain on the abdomen side section, for example, leaving holes on the dorsal side section where the fused sections have been removed. In other words, when they are peeled apart, the perimeter sections of the fused sections on the dorsal side section, for example, are torn into approximately the shapes of the fused sections while the fused sections are separated from the dorsal side section, such that the abdomen side section and the dorsal side section become separated from each other. This separation is thought to occur in the following manner. At each fused section, force by which the abdomen side section and dorsal side section become peeled apart is applied to the perimeter section of the fused section, i.e. the border section between the sheet members and the perimeter wall section, and to the perimeter wall section itself. However, since the perimeter wall section is formed by first fusing and then solidifying a plurality of sheet members, the breaking strength is stronger and tearing is less likely to occur than at the border section. Consequently, the force that causes peeling apart does not result in tearing of the fused section, i.e. the perimeter wall section that is resistant to tearing, but rather in tearing of the easily tearable border section, i.e. the perimeter section of the fused section, into the approximate shape of the fused section. Thus, the phenomenon by which the perimeter sections of the fused sections on the dorsal side section, for example, are torn into the approximate shapes of the fused sections, is thought to occur more easily when the widths of the perimeter wall sections (in the longitudinal direction or transverse direction) are basically uniform without depending on the locations surrounding the core sections.

However, in the method of Patent Literature 1, the prescribed locations that are to form the joint sections are clamped and fused by the ultrasonic horn and anvil during the short period in which the prescribed locations pass between the gap between the ultrasonic horn and anvil, and therefore the time for formation of the fused sections cannot be lengthened. Therefore, when the sheet members are fused at the protrusions of the anvil during formation of the fused sections that include perimeter wall sections, the perimeter wall sections formed by the front end portions of the protrusions are thin while the perimeter wall sections formed by the rear end portions of the protrusions are thick. In other words, it is difficult for the widths of the perimeter wall sections that are formed to be made basically uniform without depending on the locations surrounding the core sections, and the widths of the perimeter wall sections can potentially become partially thinned. When the widths of the perimeter wall sections become partially thinned, i.e. when portions of low breaking strength are present in the perimeter wall sections, the force causing them to peel apart can potentially produce tearing not at the border sections but at the portions of the perimeter wall sections that have low thickness. When this occurs, tearing proceeds from the torn portions of the perimeter wall sections, and this may lead to tearing up to the sheet members at sections other than the joint sections (for example, the front sheet or back sheet near the absorbent body). In this case, it becomes impossible to fully peel apart each of the joint sections into the abdomen side section and dorsal side section along the longitudinal direction, and in some cases the absorbent body becomes exposed or damaged between the gaps of the torn sheet members, potentially resulting in exposure or leakage of excreta.

It is therefore an object of the present invention to provide an absorbent article with joint sections, wherein it is possible to stably prevent separation of the joint sections due to movement by the wearer when the absorbent article is being worn, and to allow the abdomen side section and dorsal side section to be properly and easily separated without tearing of the sheet members, when the absorbent article is removed.

### [SOLUTION TO PROBLEM]

The absorbent article of the present invention is defined in claim 1 and is an absorbent article having a longitudinal direction, a transverse direction and a thickness direction that are mutually perpendicular, and including an abdomen side section and a dorsal side section, both edges of the abdomen side section and dorsal side section in the transverse direction overlapping in the thickness direction along the longitudinal direction and being joined together by a pair of joint sections, wherein each of the pair of joint sections includes a plurality of fused sections situated along the longitudinal direction, each of the plurality of fused sections includes: a core section where a sheet member of the abdomen side section and a sheet member of the dorsal side section are fused together in the thickness direction, and a perimeter wall section where the sheet member of the abdomen side section and the sheet member of the dorsal side section are fused together in the thickness direction, so as to surround the core section in a tubular manner in the thickness direction, and when a direction in which a difference in widths between the perimeter wall section located on both outer sides of the core section is smaller is defined as a first direction and a direction in which the difference is larger is defined as a second direction, among the longitudinal direction and the transverse direction, in the second direction, a first dimension which is a width of the perimeter wall section located on a second direction first side, i.e. one side of the core section in the second direction, is equal to or more than 40% and less than 100% of a second dimension which is a width of the perimeter wall section located on a second direction second side, i.e. the other side of the core section in the second direction.

In this absorbent article, the fused sections each have a core section and a perimeter wall section surrounding the core section in a tubular manner. That is, the perimeter wall sections can help stably prevent separation of the joint sections by movement of the wearer while the absorbent article is being worn. Moreover, at the fused sections, in the second direction in which the difference in width of the perimeter wall section is large, the width of the perimeter wall section on the second direction first side where the width is smaller (first dimension) is equal to or more than 40% and less than 100% of the width of the perimeter wall section on the second direction second side where the width is larger (second dimension). That is, it is formed so that the difference in the width of the perimeter wall section on the second direction first side and the width of the perimeter wall section on the second direction second side is not very large, but instead small. Therefore, in the fused sections also include perimeter wall sections with a small difference in the widths of the perimeter wall sections on both outer sides of the core section in the first direction, the widths of the perimeter wall sections being relatively uniform overall, without sections that are excessively thinner than the other sections. Consequently, it is possible to minimize tearing of the perimeter wall sections caused by peeling force when the abdomen side section and the dorsal side section are peeled apart, so that tearing occurs at the border sections between the sheet members and perimeter wall sections, i.e. the perimeter sections of the fused sections. As a result, the joint sections can be fully peeled apart into the abdomen side section and dorsal side section along the longitudinal direction, so that the perimeter wall sections are torn and tearing proceeds from the torn sections and tearing of the sheet member at sections other than the joint sections is minimized. Thus, the absorbent article with joint sections makes it possible to stably prevent separation of the joint sections due to movement by the wearer when the absorbent article is being worn, and to allow the abdomen side section and dorsal side section to be properly and easily separated without tearing of the sheet members, when the absorbent article is removed.

The absorbent article of the present invention is characterized in that in the first direction, widths of the perimeter wall section located on both outer sides of the core section both increase from the second direction first side toward the second direction second side.

In this absorbent article, the widths of the perimeter wall sections located on both outer sides of the core section in the first direction gradually thicken in the second direction. Therefore, the width varies in a continuous manner from the perimeter wall section of small thickness on the second direction first side in the second direction (first dimension), through the perimeter wall sections located on both outer sides of the core section in the first direction, up to the perimeter wall section of large thickness on the second direction second side in the second direction (second dimension). That is, the fused sections have gradually varying widths of the perimeter wall sections but are relatively uniform overall, and do not have sections with excessively smaller thickness than the other sections. Thus, it is possible to more stably prevent separation of the joint sections caused by movement of the wearer when the article is worn. Furthermore, it is possible to further minimize tearing of the perimeter wall sections caused by peeling force when the abdomen side section and the dorsal side section are peeled apart, so that tearing more readily occurs at the perimeter sections of the fused sections.

The absorbent article of the present invention may further comprise the features, wherein in the first direction, a third dimension, which is a width at an arbitrary location in the second direction of the perimeter wall section located on one outer side of the core section, is ±15% of a fourth dimension, which is a width at a location opposite the arbitrary location of the perimeter wall section located on the other outer side of the core section.

In this absorbent article, for the first direction, the widths of the perimeter wall sections at the arbitrary locations in the second direction, located opposite each other on both outer sides of the core section are the same within a range of ±15%. That is, the fused sections have widths of the perimeter wall sections that are more uniform overall, and further do not have sections of excessively smaller thickness than the other sections. Consequently, it is possible to further minimize tearing of the perimeter wall sections caused by peeling force when the abdomen side section and the dorsal side section are peeled apart, so that tearing more readily occurs at the perimeter sections of the fused sections.

The absorbent article of the present invention may further comprise the features, wherein a maximum dimension of the core section in the second direction is longer than a maximum dimension of the core section in the first direction.

In this absorbent article, the maximum dimension of the core section in the second direction is longer than the maximum dimension of the core section in the first direction, and therefore the fused sections have long horizontal shapes in the second direction. Therefore, compared to when the fused sections do not have horizontally long shapes, force applied to the joint sections by movement of the wearer when the article is worn can be more stably received and separation of the joint sections can be more stably prevented. Furthermore, since the fused sections are relatively long in the second direction and hence the perimeter wall sections with small differences in width at both outer sides of the core section are longer in the second direction, it is possible to further minimize the presence of sections where the widths of the perimeter wall sections are too thin. Consequently, it is possible to further minimize tearing of the perimeter wall sections caused by peeling force when the abdomen side section and the dorsal side section are peeled apart, so that tearing more readily occurs at the perimeter sections of the fused sections.

The absorbent article of the present invention may further comprise the features, wherein the first dimension of the perimeter wall sections is equal to or more than 0.5% of a maximum dimension of the core section in the second direction.

In this absorbent article, the first dimension in the second direction of the perimeter wall section (the side of smaller width) is equal to or more than 0.5% of the dimension in the second direction of the core section (which is defined as 100%), or in other words, it has a width that is equal to or more than a prescribed width (0.5% of the dimension of the core section in the second direction). Therefore, the fused sections do not have sections that are excessively thinner than the other sections in the perimeter wall sections. Consequently, it is possible to further minimize tearing of the perimeter wall sections caused by peeling force when the abdomen side section and the dorsal side section are peeled apart, so that tearing more readily occurs at the perimeter sections of the fused sections.

The absorbent article of the present invention may further comprise the features, wherein the second direction is the transverse direction, and the first dimension of the perimeter wall section located on the second direction first side of the core section at a joint section on the second direction second side among the pair of joint sections is equal to or more than 40% and less than 100% of the second dimension of the perimeter wall section located on the second direction second side of the core section at a joint section on the second direction first side among the pair of joint sections.

In this absorbent article, the first dimension of the perimeter wall section located on the second direction first side (inner side) of the core section at the joint section on the second direction second side is equal to or more than 40% and less than 100% of the second dimension of the perimeter wall section located on the second direction second side (inner side) of the core section at the joint section on the second direction first side. Therefore, the difference between the width of the perimeter wall section on the second direction first side and the width of the perimeter wall section on the second direction second side at one fused section is small, while the difference between the width of the perimeter wall section located at the second direction first side of the core section at the joint section on the second direction second side and the width of the perimeter wall section located on the second direction second side of the core section at the joint section on the second direction first side at the pair of joint sections is reduced. In other words, at the pair of joint sections, the widths of the perimeter wall sections are relatively uniform overall at the fused sections of both joint sections. Therefore, it is possible to further minimize tearing of the perimeter wall sections caused by peeling force when the abdomen side section and the dorsal side section are peeled apart, so that tearing more readily occurs at the perimeter sections of the fused sections.

The absorbent article of the present invention may further comprise the features, wherein the second direction is the transverse direction, at least a joint section on the second direction second side, of the pair of joint sections, further includes: a plurality of auxiliary fused sections arranged along the longitudinal direction on the second direction first side of the plurality of fused sections, each of the plurality of auxiliary fused sections includes: an auxiliary core section where the sheet member of the abdomen side section and the sheet member of the dorsal side section are fused together in the thickness direction, and an auxiliary perimeter wall section where the sheet member of the abdomen side section and the sheet member of the dorsal side section are fused together in the thickness direction, so as to surround the auxiliary core section in a tubular manner in the thickness direction, and an area of the auxiliary core section of each of the plurality of auxiliary fused sections is smaller than an area of the core section of each of the plurality of fused sections, in the plan view.

In this absorbent article, at least the joint section on the second direction second side further includes a plurality of auxiliary fused sections on the second direction first side of the plurality of fused sections. Therefore, compared to when the joint sections do not have a plurality of auxiliary fused sections on the second direction first side, force applied to the joint sections by movement of the wearer when the article is worn can be more stably received, and separation of the joint sections can be more stably prevented.

The absorbent article of the present invention may further comprise the features, wherein the pair of joint sections both further include the plurality of auxiliary fused sections on the second direction first side of the plurality of fused sections.

In this absorbent article, both of the pair of joint sections further include a plurality of auxiliary fused sections on the second direction first side of the plurality of fused sections. That is, at the joint section on the second direction first side, of the pair of joint sections, an auxiliary fused section is present on the side receding from a longitudinal center line (imaginary line) running in the longitudinal direction through the center of the absorbent article in the transverse direction, i.e. on the outer side in the transverse direction, with respect to the fused sections. At the same time, at the joint section on the second direction second side, of the pair of joint sections, an auxiliary fused section is present on the side approaching the longitudinal center line, i.e. the inner side in the transverse direction, with respect to the fused section. In the pair of joint sections, therefore, the fused sections and auxiliary fused sections can be formed with asymmetry with respect to the longitudinal center line. Therefore, by having the relatively small auxiliary fused section on the outer side in the transverse direction at the joint section on the second direction first side, it is possible for the section on the outer side in the transverse direction to be easily deformed (easily rounded) inward. On the other hand, by having the relatively large auxiliary fused section on the outer side in the transverse direction at the joint section on the second direction second side, it is possible for the section on the outer side in the transverse direction to be less easily deformed (less easily rounded) inward. As a result, at the pair of joint sections it is possible to produce right-left asymmetry for not only the arrangement of the fused sections and auxiliary fused sections but also their overall shapes (degree of rounding). Thus, by visual and/or tactile confirmation of the arrangement of the fused sections and auxiliary fused sections and the overall shapes and asymmetry of the joint sections, it is possible to easily distinguish left and right in the transverse direction of the absorbent article. Therefore, the thickness direction regions, i.e. the abdomen side section and dorsal side section of the absorbent article, can be easily recognized and distinguished (when determining the right/left relationships of the arrangement or shapes in advance while the absorbent article is in the package).

The absorbent article of the present invention may further comprise the features, wherein a shape of the core section of each of the plurality of auxiliary fused sections are circular, in the plan view.

In this absorbent article, the shapes of the core sections of the auxiliary fused sections are circular, and therefore the shapes of the auxiliary fused sections including the perimeter wall sections are also circular. Therefore, force applied to the joint sections from different directions, by movement of the wearer when the article is worn, can be more stably received and separation of the joint sections can be more stably prevented.

The absorbent article of the present invention may further comprise the features, wherein the second direction is the transverse direction, the absorbent article has a longitudinal center line running in the longitudinal direction through a center in the transverse direction of the absorbent article, and the plurality of fused sections at the pair of joint sections are disposed along oblique directions with respect to the longitudinal direction, gradually receding toward outer sides in the transverse direction from a waist side toward a leg side or from the leg side toward the waist side, of the absorbent article in the longitudinal direction, with respect to the longitudinal center line.

In this absorbent article, the plurality of fused sections of the pair of joint sections are disposed along oblique directions with respect to the longitudinal direction, so as to gradually recede from the waist side toward the leg side or from the leg side toward the waist side, with respect to the longitudinal center line (imaginary line). In other words, the plurality of fused sections of the pair of joint sections are disposed in a divergent shape from the waist side toward the leg side or from the leg side toward the waist side. Consequently, in the transverse direction of the waist side or leg side, the portions further on the outer sides than the plurality of fused sections at the joint sections (the portions further on the outer sides than the fused sections with respect to the longitudinal center line) form protrusions (ears) that protrude toward the outer sides. Thus, when the wearer removes the absorbent article, the protrusions are gripped and pulled to the outer side in the transverse direction, thereby allowing a gap to be formed between the portion on the waist side or leg side of the absorbent article and the body, to facilitate insertion of the finger. Alternatively, it may facilitate insertion of a finger between the body and the portion of the leg side or waist side opposite the protrusion in the longitudinal direction of the absorbent article. Thus, a finger can be inserted into the gap, allowing the abdomen side section and dorsal side section to be easily peeled apart.

A method for producing an absorbent article of the present invention is defined in claim 10 and is a method of producing an absorbent article, the method including a first forming step of, while transporting a continuous sheet members, in which semi-products of the absorbent article wherein the abdomen side section and dorsal side section are laminated together but not joined are continuously aligned in the transverse direction, with the transverse direction as a machine direction, forming each of the plurality of fused sections in order with a time difference at locations corresponding to one of the pair of joint sections in the semi-product; and a second forming step of forming each of the plurality of fused sections in order with a time difference at locations corresponding to the other of the pair of joint sections in the semi-product, after the first forming step.

In this method for producing an absorbent article, a plurality of fused sections are formed in order with a time difference when the joint sections are formed. It is thereby possible to reduce the number of fused sections formed per unit time, which are gripped and formed by the ultrasonic horn and anvil that form the fused sections. As a result, the gripping pressure (linear pressure) applied to each fused section can be increased. This allows the fused sections to be stably formed, and can produce more uniform and adequate widths for the perimeter wall sections. Consequently, it is possible to further minimize tearing of the perimeter wall sections caused by peeling force when the abdomen side section and the dorsal side section are peeled apart, so that tearing more readily occurs at the perimeter sections of the fused sections.

### [ADVANTAGEOUS EFFECTS OF INVENTION]

In an absorbent article with joint sections according to the present invention, it is possible to stably prevent separation of the joint sections due to movement by the wearer when the absorbent article is being worn, and to allow the abdomen side section and dorsal side section to be properly and easily separated without tearing of the sheet members, when the absorbent article is removed.

### BRIEF DESCRIPTION OF DRAWINGS

Fig. 1 is a perspective view showing an example of a construction of a disposable diaper according to an embodiment.
Fig. 2 is a plan view of the disposable diaper of Fig. 1 in an expanded state.
Fig. 3 is a partial enlarged plan view showing an example of a construction of joint sections of the disposable diaper of Fig. 1.
Fig. 4 is a schematic view showing an example of a construction of a fused section of the joint of Fig. 3.
Fig. 5 is a schematic view showing an example of a construction of a cover sheet at the joint section of Fig. 3.
Fig. 6 is a schematic view illustrating functions of the disposable diaper according to the embodiment.
Fig. 7 is a schematic view showing an example of a construction of a fusing apparatus according to the embodiment.
Fig. 8 is a schematic view showing an example of a construction of protrusion rows on an anvil of the fusing apparatus of Fig. 7.
Fig. 9 is a perspective view showing an example of a method for producing the disposable diaper according to the embodiment.
Fig. 10 is a schematic view showing a process of forming the joint sections in the production method of Fig. 9.

### DESCRIPTION OF EMBODIMENTS

An absorbent article according to an embodiment of the present invention will now be explained using a pants-type disposable diaper (hereunder also referred to simply as "disposable diaper") as an example. However, the absorbent article of the present invention is not limited to this example, and the present invention may be applied to various types of absorbent articles without departing from a scope of the present invention. The absorbent article may be a so-called 3-piece type or 2-piece type disposable diaper.

A disposable diaper 1 according to an embodiment of the present invention will be described first.

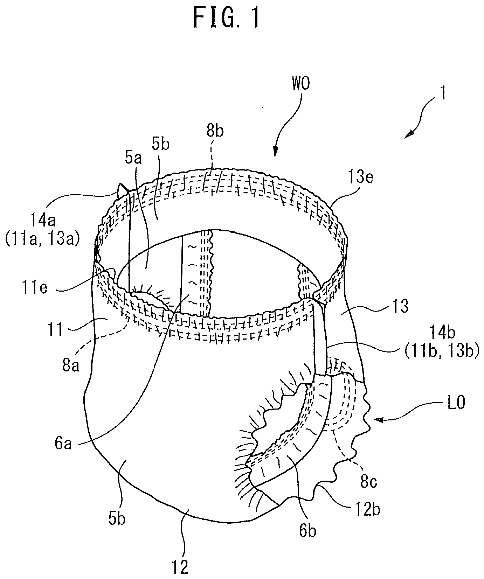

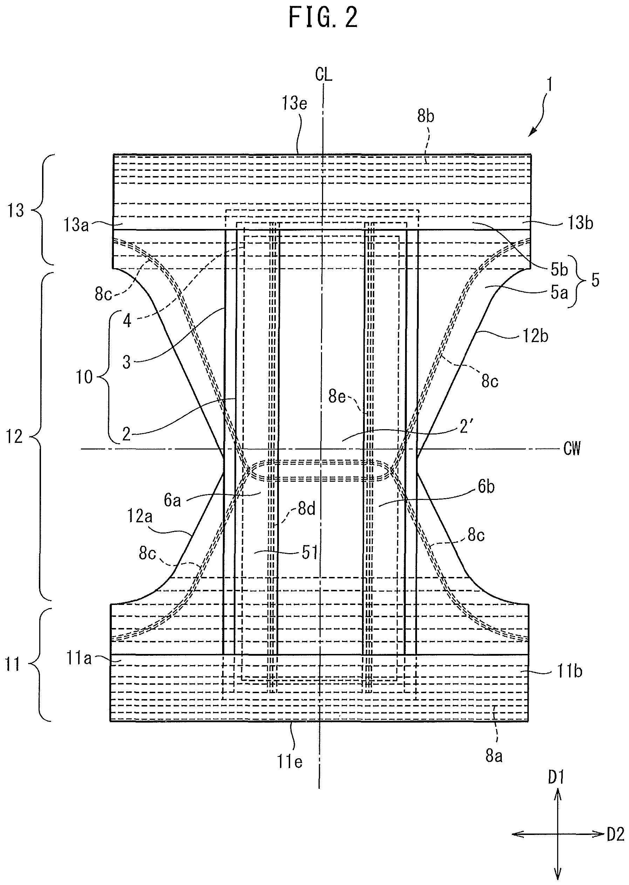

Fig. 1 and Fig. 2 are views showing examples of the construction of the disposable diaper 1 according to this embodiment. Fig. 1 is a perspective view showing the state of the disposable diaper 1 when it is used, and Fig. 2 is a plan view showing the state of the disposable diaper 1 when it is expanded. In the state shown in Fig. 2, the disposable diaper 1 has a longitudinal direction D1, a transverse direction D2 and a thickness direction D3 that are mutually perpendicular. The disposable diaper 1 has a longitudinal center line CL running in the longitudinal direction D1 through the center in the transverse direction D2, and a widthwise center line CW running in the transverse direction D2 through the center in the longitudinal direction D1. The directions toward and the directions away from the longitudinal center line CL are the directions toward the inner sides and the directions toward the outer sides, respectively, in the transverse direction D2. The directions toward and the directions away from the widthwise center line CW are the directions toward the inner sides and the directions toward the outer sides, respectively, in the longitudinal direction D1. The view of the disposable diaper 1 from above in the thickness direction D3, when it is placed flat surface containing the longitudinal direction D1 and transverse direction D2, will be referred to as the "plan view", and the shape as seen in the plan view will be referred to as the "planar shape". The terms "skin side" and "non-skin side" refer, respectively, to the side relatively near the skin and the side relatively far from the skin of the wearer in the thickness direction D3 of the disposable diaper 1 when the disposable diaper 1 is worn. These directions also apply when the disposable diaper 1 of Fig. 1 is in the flat state before use, i.e. in the state where the disposable diaper 1 of Fig. 2 is folded on the folding line along the widthwise center line CW.

The disposable diaper 1 is a pants-type diaper including an abdomen side section 11, a dorsal side section 13, and a crotch section 12 between the abdomen side section 11 and dorsal side section 13. The abdomen side section 11 is the portion of the disposable diaper 1 that contacts the abdominal region of the wearer. The crotch section 12 is the portion of the disposable diaper 1 that contacts the crotch of the wearer. The dorsal side section 13 is the portion of the disposable diaper 1 that contacts the gluteal region and/or dorsal region of the wearer. Both edge sections 11a, 11b in the transverse direction D2 of the abdomen side section 11 and both edge sections 13a, 13b in the transverse direction D2 of the dorsal side section 13 are joined together at a pair of joint sections 14a, 14b while overlapping in the thickness direction D3 along the longitudinal direction D1. In the disposable diaper 1, a waist opening WO is formed by the end section 11e on the opposite side of the abdomen side section 11 from the crotch section 12 in the longitudinal direction D1 and the end section 13e on the opposite side of the dorsal side section 13 from the crotch section 12 in the longitudinal direction D1. A pair of leg openings LO, LO are also formed in the disposable diaper 1 by both side sections 12a, 12b of the crotch section 12 in the transverse direction D2.

The disposable diaper 1 includes an absorbent main body 10. The absorbent main body 10 includes a liquid-permeable top sheet 2, a liquid-impermeable back sheet 3, and an absorbent body 4 situated between the top sheet 2 and back sheet 3. The top sheet 2 may be, for example, a liquid-permeable nonwoven fabric or woven fabric, a liquid-permeable pore-formed synthetic resin film, or a composite sheet thereof. The back sheet 3 may be, for example, a liquid-impermeable nonwoven fabric or synthetic resin film, a composite sheet thereof, an SMS nonwoven fabric, or the like. The absorbent body 4 may be pulp fibers, synthetic fibers or absorbing polymers, for example. The absorbent body 4 and the top sheet 2 and back sheet 3 are each joined with an adhesive, the top sheet 2 and back sheet 3 being joined by an adhesive at their peripheries. The adhesive used may be a publicly known material such as a hot-melt adhesive, for example.

The disposable diaper 1 further includes a liquid-impermeable cover sheet 5. For this embodiment, the cover sheet 5 includes a cover sheet 5a situated on the skin side and a cover sheet 5b situated on the non-skin side, which are laminated together in the thickness direction D3 and joined by an adhesive or the like. For this embodiment, both edges of the cover sheet 5b in the longitudinal direction D1 are folded over to the skin side so as to cover both edges of the cover sheet 5a in the longitudinal direction D1. The cover sheets 5b, 5b at the folded locations on the abdomen side section 11 and dorsal side section 13 form the end section 11e of the abdomen side section 11 and the end section 13e of the dorsal side section 13, respectively. On the skin side surface of the cover sheet 5, the absorbent main body 10 is placed with the top sheet 2 facing the skin side. In the disposable diaper 1, the non-skin side surface of the disposable diaper 1, i.e. the outer side surface, is formed by the cover sheet 5b, and the skin side surface of the disposable diaper 1, i.e. the inner side surface, is formed by the top sheet 2 and the cover sheet 5b at the end section 11e and end section 13e. The cover sheet 5 may be any desired liquid-impermeable sheet, such as, for example, a liquid-impermeable nonwoven fabric or synthetic resin film, a composite sheet thereof, or an SB nonwoven fabric or SMS nonwoven fabric. Examples of materials for the cover sheet 5 include polyolefin-based materials such as polypropylene or polyethylene. The basis weight of the cover sheet 5 may be 5 to 100 g/m², for example, and it is preferably 10 to 50 g/m². The dimension of the cover sheet 5 in the thickness direction D3 (thickness) may be 0.2 to 5 mm, for example, and it is preferably 0.2 to 2 mm. The cover sheet 5 may be a single sheet, and it does not need to be folded.

The disposable diaper 1 may further includes a liquid-impermeable pair of anti-leakage walls 6a, 6b and elastic members 8 (8a, 8b, 8c, 8d, 8e). The pair of anti-leakage walls 6a, 6b are arranged along the longitudinal direction D1, on both sides of the center section 2' of the top sheet 2 in the transverse direction D2. The elastic member 8a and elastic member 8b extend in the transverse direction D2 between the cover sheet 5a and cover sheet 5b on the abdomen side section 11 and dorsal side section 13, respectively, and are situated across and held at an interval in the longitudinal direction D1. The elastic members 8a, 8b allow expansion and contraction of the waist opening WO. The elastic members 8c are disposed in a continuous manner largely along the longitudinal direction D1, at both edges in the transverse direction D2 of the portion of the crotch section 12 on the side of the dorsal side section 13, and along the transverse direction D2 at the center section of the crotch section 12. The elastic members 8c allow expansion and contraction of the pair of leg openings LO, LO, respectively. At the inside ends of the pair of anti-leakage walls 6a, 6b in the transverse direction D2, there are disposed an elastic member 8d and an elastic member 8e, each extending in the longitudinal direction D1. The elastic members 8d, 8e allow expansion and contraction of the anti-leakage walls 6a, 6b, respectively. Rubber thread is an example for the elastic members 8.

The construction of each of the pair of joint sections 14a, 14b will now be described.

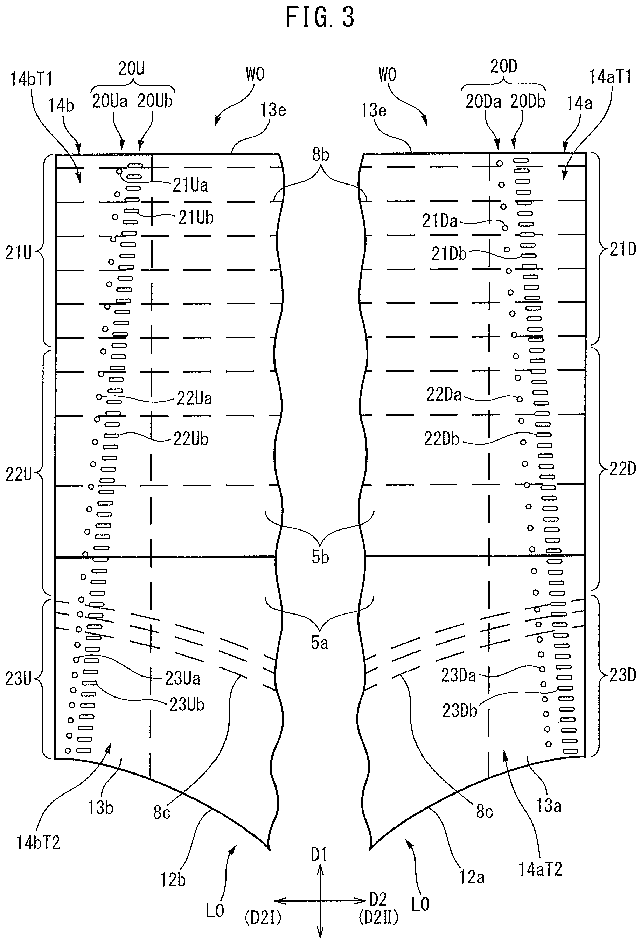

Fig. 3 is a partial enlarged plan view schematically showing examples of the constructions of the pair of joint sections 14a, 14b of the disposable diaper 1. Fig. 3 shows the joint sections 14a, 14b of the disposable diaper 1 of Fig. 1 as seen from the dorsal side section 13. In Fig. 3, the approximate center section in the transverse direction D2 of the disposable diaper 1 is omitted. The joint section 14b is disposed on the left side as seen from the dorsal side section 13 (one side in the transverse direction D2, i.e. a transverse direction first side D2I), while the joint section 14a is disposed on the right side as seen from the dorsal side section 13 (the other side in the transverse direction D2, i.e. a transverse direction second side D2II). The joint section 14a is formed by having the end section 11a in the transverse direction D2 of the abdomen side section 11 and the end section 13a in the transverse direction D2 of the dorsal side section 13 overlapping in the thickness direction D3 along the longitudinal direction D1, and fused and joined at least at a plurality of locations. Similarly, the joint section 14b is formed by having the end section 11b in the transverse direction D2 of the abdomen side section 11 and the end section 13b in the transverse direction D2 of the dorsal side section 13 overlapping in the thickness direction D3 along the longitudinal direction D1, and fused and joined at a plurality of locations. There are no particular restrictions on the respective dimensions of the joint sections 14a, 14b in the longitudinal direction D1 and transverse direction D2, and they may be 50 to 250 mm and 3 to 20 mm, for example, respectively. In addition to fusion, the pair of joint sections 14a, 14b may also be joined totally or partially by an adhesive (for example, a hot-melt adhesive).

The joint sections 14a, 14b include joining rows 20D, 20U where a plurality of locations that are fused, i.e. fused sections, are disposed in a manner aligned in a row along the longitudinal direction D1 across intervals. Thus, the joining row 20D joins together the end section 11a and the end section 13a, while the joining row 20U joins together the end section 11b and the end section 13b. The overall arrangement of the joining row 20D and joining row 20U is symmetrical with respect to the longitudinal center line CL. However, considering the arrangements of the plurality of fused sections, the joining row 20D and joining row 20U are asymmetrical with respect to the longitudinal center line CL, and therefore the pair of joint sections 14a, 14b are asymmetrical with respect to the longitudinal center line CL.

For this embodiment, the joint section 14a has, in the longitudinal direction D1, a waist side region 21D including the end on the waist opening WO side, a leg side region 23D including the end on the leg opening LO side, and a center region 22D between the two regions. Similarly, the joint section 14b has, in the longitudinal direction D1, a waist side region 21U including the end on the waist opening WO side, a leg side region 23U including the end on the leg opening LO side, and a center region 22U between the two regions. The waist side regions 21D, 21U and the leg side regions 23D, 23U are, for example, the regions in a range of 1/8 to 1/3 of the joint sections from the ends of the waist opening WO side and leg opening LO side, respectively, in the longitudinal direction D1, while the center regions 22D, 22U are the remaining regions. However, the waist side regions 21D, 21U and leg side regions 23D, 23U do not necessarily have to have identical lengths in the longitudinal direction D1. For this embodiment, the waist side regions 21D, 21U, center regions 22D, 22U and leg side regions 23D, 23U are regions in ranges of approximately 3/10, 4/10, 3/10 of the joints.

For this embodiment, the joining row 20D of the joint section 14a includes an auxiliary joining row 20Da and a main joining row 20Db. The auxiliary joining row 20Da is situated on one side in the transverse direction D2 (the left side in Fig. 3; the second direction first side D2I), and extends in the longitudinal direction D1. The auxiliary joining row 20Da includes a plurality of auxiliary fused sections 21Da, a plurality of auxiliary fused sections 22Da and a plurality of auxiliary fused sections 23Da, which are aligned along the longitudinal direction D1, in the waist side region 21D, center region 22D and leg side region 23D, respectively. The main joining row 20Db, on the other hand, is situated on the other side in the transverse direction D2 (the right side in Fig. 3; the second direction second side D2II), and extends in the longitudinal direction D1. The main joining row 20Db includes a plurality of fused sections 21Db, a plurality of fused sections 22Db and a plurality of fused sections 23Db, which are aligned along the longitudinal direction D1, in the waist side region 21D, center region 22D and leg side region 23D, respectively. For this embodiment, the structures of the plurality of auxiliary fused sections 21Da are the same, but alternatively one or more may differ. This also applies to the plurality of auxiliary fused sections 22Da, 23Da. Also for this embodiment, the structures of the plurality of fused sections 21Db are the same, but alternatively one or more may differ. This also applies to the plurality of fused sections 22Db, 23Db. Some or all of the plurality of auxiliary fused sections 21Da, 22Da, 23Da may also be absent.

For this embodiment, the joining row 20U of the joint section 14b also includes another auxiliary joining row 20Ua and another main joining row 20Ub. The other auxiliary joining row 20Ua is situated on one side in the transverse direction D2 (the left side in Fig. 3; the second direction first side D2I), and extends in the longitudinal direction D1. The other auxiliary joining row 20Ua includes a plurality of other auxiliary fused sections 21Ua, a plurality of other auxiliary fused sections 22Ua and a plurality of other auxiliary fused sections 23Ua, that are aligned along the longitudinal direction D1, in the waist side region 21U, center region 22U and leg side region 23U, respectively. The other main joining row 20Ub, on the other hand, is situated on the other side in the transverse direction D2 (the right side in Fig. 3; the second direction second side D2II), and extends in the longitudinal direction D1. The other main joining row 20Ub includes a plurality of other fused sections 21Ub, a plurality of other fused sections 22Ub and a plurality of other fused sections 23Ub, which are aligned along the longitudinal direction D1, in the waist side region 21U, center region 22U and leg side region 23U, respectively. For this embodiment, the structures of the plurality of other auxiliary fused sections 21Ua are the same, but alternatively one or more may differ. This also applies to the plurality of other auxiliary fused sections 22Ua, 23Ua. Moreover, for this embodiment, the structures of the plurality of other fused sections 21Ub are the same, but alternatively one or more may differ. This also applies to the plurality of other fused sections 22Ub, 23Ub. Some or all of the plurality of other auxiliary fused sections 21Ua, 22Ua, 23Ua may also be absent.

The planar shapes and mutual intervals (in the longitudinal direction D1) of the fused sections 21Db to 23Db and 21Ub to 23Ub and the auxiliary fused sections 21Da to 23Da and 21Ua to 23Ua are not particularly restricted so long as the prescribed strength as joint sections can be obtained. The shapes may be circular, oblong (rounded rectangular), elliptical, polygonal, star-shaped or linear, for example. For this embodiment, the planar shapes of the fused sections 21Db to 23Db and 21Ub to 23Ub are long oblong (rounded rectangular) in the transverse direction D2 and their sizes are mutually identical, while the planar shapes of the auxiliary fused sections 21Da to 23Da and 21Ua to 23Ua are circular, and their sizes are mutually identical. Their mutual intervals are the same between the adjacent fused sections, and the same between the adjacent auxiliary fused sections. One or more of the fused sections may differ in shape, size or interval from one or more of the others, and one or more of the auxiliary fused sections may also differ in shape, size or interval from one or more of the others.

Each of the plurality of auxiliary fused sections 21Da to 23Da has smaller area and shorter outer peripheral length, than each of the plurality of fused sections 21Db to 23Db. For example, the auxiliary fused sections 21Da have smaller areas than the fused sections 21Db, and preferably the auxiliary fused sections 22Da have smaller areas than the fused sections 22Db, and preferably the auxiliary fused sections 23Da have smaller areas than the fused sections 23Db. The auxiliary fused sections 23Da preferably have smaller areas than the fused sections 21Db. These relationships may also apply to the outer peripheral lengths. Therefore, each of the plurality of auxiliary fused sections 21Da to 23Da has lower engaging strength than each of the plurality of fused sections 21Db to 23Db. When the shapes are circular, the sizes of each of the auxiliary fused sections 21Da to 23Da may be 0.5 to 5 mm diameters, for example. If the auxiliary fused sections are smaller than 0.5 mm, temporary fastening protrusions for formation of the auxiliary fused sections, provided on the anvil during production, will be too fine and will tend to be abraded off, potentially making it impossible to form the auxiliary fused sections in a stable manner. If the auxiliary fused sections are larger than 5 mm, then when the disposable diaper 1 is removed after use, it will be necessary to peel apart both the large auxiliary fused sections and the fused sections, and they may potentially be very difficult to peel apart. When the shapes are long oblong (rounded rectangular) in the transverse direction D2, the sizes of the fused sections 21Db to 23Db may have short sides (short diameters) of 0.5 to 5 mm and long sides (long diameters) of 1 to 20 mm, for example. The distances between the adjacent auxiliary joining row 20Da and main joining row 20Db may be 1 to 10 mm, for example, the distance between the adjacent auxiliary fused sections may be 1 to 30 mm, for example, and the distance between adjacent fused sections may be 1 to 10 mm, for example. The intervals between adjacent fused sections may be constant or varying based on location. The intervals between the auxiliary fused sections and fused sections may be 1 to 30 mm, for example. If the interval is less than 1 mm, the auxiliary fused sections and fused sections will be too close, causing their outer peripheries to overlap and resulting in connection between the auxiliary fused sections and fused sections, which may potentially make it very difficult to achieve peeling apart when the disposable diaper 1 is removed after use. If the intervals are greater than 30 mm, on the other hand, the engaging strength will be too weak, potentially causing the joint sections to detach when worn.

For this embodiment, the construction of each of the plurality of other auxiliary fused sections 21Ua to 23Ua and each of the plurality of other fused sections 21Ub to 23Ub are the same as the construction of each of the plurality of other auxiliary fused sections 21Da to 23Da and each of the plurality of other fused sections 21Db to 23Db, respectively.

The joining rows 20D, 20U in each region of this embodiment are both double rows. However, the joining rows 20D, 20U may both be single rows or more than double rows, and the number of rows may also differ between the regions. The number of regions for the joining rows may be one, two, or 4 or more.

Considering the arrangement of the plurality of fused sections, as mentioned above, the joining rows 20D and joining rows 20U are asymmetrical with respect to the longitudinal center line CL. Thus, the auxiliary joining row 20Da and main joining row 20Db, and the other auxiliary joining row 20Ua and other main joining row 20Ub, are asymmetrical with respect to the longitudinal center line CL. Therefore, the plurality of auxiliary fused sections 21Da to 23Da and plurality of fused sections 21Db to 23Db and the plurality of other auxiliary fused sections 21Ua to 23Ua and plurality of other fused sections 21Ub to 23Ub are asymmetrical with respect to the longitudinal center line CL.

For this embodiment, the joining rows 20D, 20U of the pair of joint sections 14a, 14b are disposed along oblique directions in the longitudinal direction D1, in such a manner as to gradually recede in the transverse direction D2 with respect to the longitudinal center line CL, from the waist opening WO side toward the leg opening LO side in the longitudinal direction D1. That is, at the joint section 14a, the plurality of fused sections 21Db to 23Db are disposed along an oblique direction with respect to the longitudinal direction D1, in such a manner as to gradually recede outward in the transverse direction D2 (the transverse direction second side D2II) with respect to the longitudinal center line CL, from the waist opening WO side toward the leg opening LO side in the longitudinal direction D1. At the same time, the plurality of auxiliary fused sections 21Da to 23Da are likewise disposed along an oblique direction with respect to the longitudinal direction D1. Similarly, at the joint section 14b, the plurality of fused sections 21Ub to 23Ub are disposed along an oblique direction with respect to the longitudinal direction D1, in such a manner as to gradually recede outward in the transverse direction D2 (the transverse direction first side D2I) with respect to the longitudinal center line CL, from the waist opening WO side toward the leg opening LO side in the longitudinal direction D1. At the same time, the plurality of auxiliary fused sections 21Ua to 23Ua are likewise disposed along an oblique direction with respect to the longitudinal direction D1. Therefore, the (plurality of fused sections and plurality of auxiliary fused sections of the) joining rows 20D, 20U of the pair of joint sections 14a, 14b are formed in an approximately divergent manner, spreading out from the waist opening WO side toward the leg opening LO side (forming a shape similar to a fan shape), in the plan view. The angle formed by them is preferably 0 to 20 degrees and more preferably 2 to 10 degrees, from the viewpoint of compatibility with the body of the wearer.

According to another embodiment of the present invention, the plurality of fused sections and plurality of auxiliary fused sections of the pair of joint sections 14a, 14b may be disposed along oblique directions in the longitudinal direction D1, in such a manner as to gradually recede outward in the transverse direction D2 with respect to the longitudinal center line CL, from the leg opening LO side toward the waist opening WO side in the longitudinal direction D1. That is, the (plurality of fused sections and plurality of auxiliary fused sections of the) joining rows 20D, 20U of the pair of joint sections 14a, 14b may be formed in an approximately divergent manner, spreading out from the leg opening LO side toward the waist opening WO side (forming a shape similar to a fan shape), in the plan view.

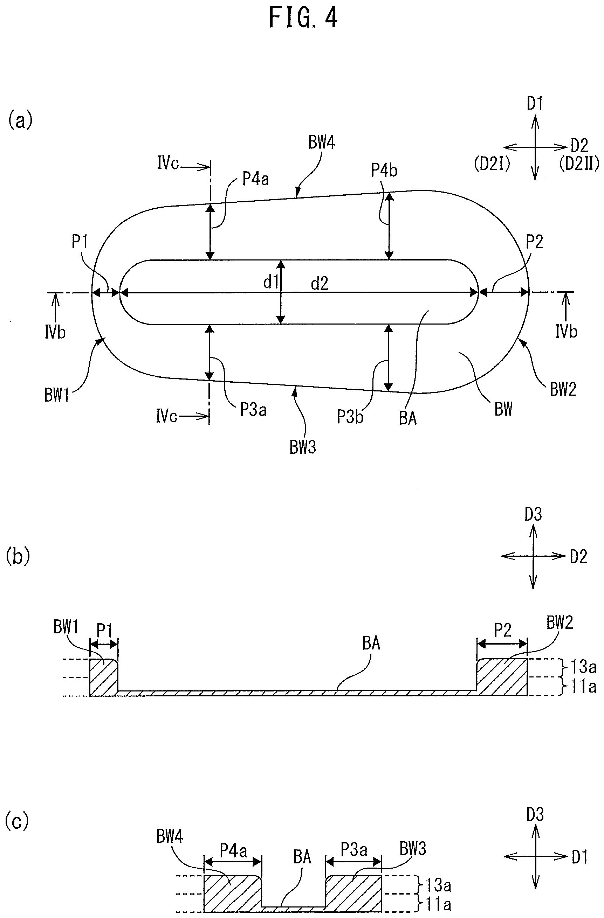

Fig. 4 is a schematic view showing an example of the construction of a fused section of the pair of joint sections 14a, 14b. Fig. 4(a) is a diagram of the fused section as seen from the thickness direction D3, Fig. 4(b) is a cross-sectional view on IVb-IVb of Fig. 4(a), and Fig. 4(c) is a cross-sectional view on IVc-IVc of Fig. 4(a). The fused sections (21Db to 23Db and 21Ub to 23Ub) each have a core section BA and a perimeter wall section BW. The core section BA is the section where the sheet member of the abdomen side section 11 and the sheet member on the dorsal side section 13 are pressed by the protrusion of the anvil during formation of the fused section, and fused together in the thickness direction D3. The perimeter wall section BW is the section where the sheet member of the abdomen side section 11 and the sheet member on the dorsal side section 13 are fused together in the thickness direction D3 around the protrusion of the anvil during formation of the fused section, so as to surround the core section BA in a tubular fashion in the thickness direction D3. In other words, the perimeter wall section BW has a shape protruding in the thickness direction T in a manner surrounding the core section BA. The angle formed between the base surface of the core section and the side surface of the perimeter wall section BW is between 45° and 135°, preferably between 60° and 120° and more preferably between 80° and 100°, with respect to the thickness direction T. The core section BA is thinner in the thickness direction D3 while the perimeter wall section BW is thicker in the thickness direction D3. The engaging strength of the fused section mainly depends on the size of the area of the fused section as seen from the thickness direction D3, and therefore it depends largely on the area of the core section BA and perimeter wall section BW. The engaging strength is stronger when the size of the area of the core section BA or perimeter wall section BW is larger. The area of the perimeter wall section BW is greater when the width of the perimeter wall section BW or the outer peripheral length of the core section BA is larger. The construction of each fused section may differ in shape, but the aforementioned basic construction of having the core section BA and perimeter wall section BW is the same. Incidentally, the construction of each auxiliary fused section (21Da to 23Da and 21Ua to 23Ua) may also differ in shape, but the aforementioned basic construction of the fused section that has the core section BA and perimeter wall section BW is the same. Therefore, the construction of each auxiliary fused section (21Da to 23Da and 21Ua to 23Ua) will not be explained.

For this embodiment, as shown in Fig. 4(a), the perimeter wall section BW includes a first perimeter wall section BW1 and second perimeter wall section BW2 located on both outer sides of the core section BA in the transverse direction D2, and a third perimeter wall section BW3 and fourth perimeter wall section BW4 located on both outer sides of the core section BA in the longitudinal direction D1. The perimeter wall section BW has a structure wherein the first perimeter wall section BW1, third perimeter wall section BW3, second perimeter wall section BW2 and fourth perimeter wall section BW4 (and first perimeter wall section BW1) are mutually connected in that order, set in the thickness direction D3 in a tubular (annular) manner from the perimeter edge of the core section BA. In this embodiment, the borders between the first perimeter wall section BW1 and second perimeter wall section BW2 and the third perimeter wall section BW3 and fourth perimeter wall section BW4 are parallel lines in the longitudinal direction D1. The portions that are in contact with the straight linear portion of the core section BA are the third perimeter wall section BW3 and fourth perimeter wall section BW4.

For either the longitudinal direction D1 or the transverse direction D2, the direction where the difference in width of each fused section (21Db to 23Db and 21Ub to 23Ub) between the perimeter wall sections BW located on both outer sides of the core section BA is smaller is supposed to be the first direction, and the direction where it is larger is supposed to be the second direction. However, for the first perimeter wall section BW1 and second perimeter wall section BW2, the "width" is the thickness of the perimeter wall section in the transverse direction D2, and for the third perimeter wall section BW3 and fourth perimeter wall section BW4, it is the thickness of the perimeter wall section in the longitudinal direction D1. In the second direction, the fused section is constructed so that the first dimension, which is the width of the perimeter wall section BW located on one side in the second direction of the core section BA, is equal to or more than 40% and less than 100% of the second dimension, which is the width of the perimeter wall section BW located on the other side in the second direction of the core section BA. That is, the difference between the first dimension and second dimension is minimized to a fixed range.

For this embodiment, therefore, the difference in the widths of the first perimeter wall section BW1 and the second perimeter wall section BW2 located on both outer sides of the core section BA in the transverse direction D2 is larger than the difference in the widths of the third perimeter wall section BW3 and fourth perimeter wall section BW4 located on both outer sides of the core section BA in the longitudinal direction D1. The transverse direction D2 may therefore be considered to be the second direction, and the longitudinal direction D1 to be the first direction. For this embodiment, therefore, as shown in Fig. 4(a) and (b), in the transverse direction D2 (second direction), the first dimension PI, which is the width of the first perimeter wall section BW1 (perimeter wall section) located on the transverse direction first side D2I (second direction first side), as one side in the transverse direction D2 of the core section BA, is equal to or more than 40% and less than 100% of the second dimension P2, which is the width of the second perimeter wall section BW2 (perimeter wall section) located on the transverse direction second side D2II (second direction second side), as the other side in the transverse direction D2 of the core section BA. That is, the difference in widths of the third perimeter wall section BW3 and fourth perimeter wall section BW4 is small while the difference in widths of the first perimeter wall section BW1 and second perimeter wall section BW2 are limited to a prescribed range, so that the width of the perimeter wall section BW of the fused section is relatively uniform overall.

According to the invention, the widths of the perimeter wall sections BW located on both outer sides of the core section BA, in the first direction, both increase from the second direction first side toward the second direction second side. For this embodiment, therefore, as shown in Fig. 4(a), the widths of the third perimeter wall section BW3 and fourth perimeter wall section BW4 located on both outer sides of the core section BA, in the longitudinal direction D1, both increase from the transverse direction first side D2I toward the transverse direction second side D2II. Specifically, as regards the third dimension P3 which is the width of the third perimeter wall section BW3, the third dimension P3b on the transverse direction second side D2II is larger than the third dimension P3a on the transverse direction first side D2I. Similarly, as regards the fourth dimension P4 which is the width of the fourth perimeter wall section BW4, the fourth dimension P4b on the transverse direction second side D2II is larger than the fourth dimension P4a on the transverse direction first side D2I. This may also be considered to be a feature of the disposable diaper 1 when it is produced using any of the production methods described below.

For this embodiment, moreover, in the first direction, the third dimension, which is the width at an arbitrary location in the second direction of the perimeter wall section BW located on one outer side of the core section BA, is ±15% of the size of the fourth dimension, which is the width at a location opposite the arbitrary location of the perimeter wall section BW located on the other outer side of the core section BA. Therefore, for this embodiment, as shown in Fig. 4(a) and (c), in the longitudinal direction D1, the third dimensions P3a, P3b, which are the widths at arbitrary locations in the transverse direction D2 of the third perimeter wall section BW3 located on one outer side of the core section BA, are ±15% of the sizes of the fourth dimensions P4a, P4b, which are the widths at locations opposite the arbitrary locations of the fourth perimeter wall section BW4 located on the other outer side of the core section BA. In other words, 0.85 × P4a ≤ P3a ≤ 1.15 × P4a, and 0.85 × P4b ≤ P3b ≤ 1.15 × P4b. Therefore, the third dimension P3 and fourth dimension P4 are approximately the identical sizes.

Moreover, for this embodiment, as shown in Fig. 4(a), the maximum dimension d2 in the transverse direction D2 (second direction) of the core section BA is longer than the maximum dimension d1 in the longitudinal direction D1 (first direction) of the core section BA. That is, each fused section 21Db is formed in a horizontally long fashion. Here, it is a rounded rectangular shape, for example. Furthermore, for this embodiment, the first dimension P1 of the perimeter wall section BW is equal to or more than 0.5% of the maximum dimension d2 in the transverse direction D2 (second direction) of the core section BA. In other words, 0.5 ≤ (P1/d2) × 100 (%). Consequently, the first dimension PI, which is the smallest dimension of the perimeter wall section BW, is equal to or more than 0.5% of the maximum dimension d2 of the core section BA. The width of the perimeter wall section BW is therefore a dimension that is at least a prescribed width overall.

For this embodiment, the first dimension P1 of the first perimeter wall section BW1, located on the transverse direction first side D2I of the core section BA at the joint section 14a on transverse direction second side D2II among the pair of joint sections 14a, 14b, is equal to or more than 40% and less than 100% of the second dimension P2 of the second perimeter wall section BW2, located on the transverse direction second side D2II of the core section BA at the joint section 14b on transverse direction first side D2I among the pair of joint sections 14b. That is, 0.4 × (second dimension P2 of joint section 14b) ≤ (first dimension P1 of joint section 14a) < 1 × (second dimension P2 of joint section 14b). Therefore, the width of the perimeter wall section BW of the fused section is relatively uniform for the pair of joint sections 14a, 14b as a whole.

The planar shapes of the core sections BA of the auxiliary fused sections 21Da to 23Da and 21Ua to 23Ua and fused sections 21Db to 23Db and 21Ub to 23Ub are not particularly restricted, and may be circular, oblong, (rounded rectangular), elliptical, polygonal, star-shaped or linear, for example. For example, the planar shapes of the core sections BA of the auxiliary fused sections 21Da to 23Da and 21Ua to 23Ua may be circular. The shapes of the auxiliary fused sections 21Da to 23Da and 21Ua to 23Ua may be shapes approximately corresponding to the shapes of the core sections BA (the core sections BA being smaller). The planar shapes of the core sections BA of the fused sections 21Db to 23Db and 21Ub to 23Ub, on the other hand, may be long oblong (rounded rectangular) shapes in the transverse direction D2. The shapes of the fused sections 21Db to 23Db and 21Ub to 23Ub may be shapes approximately corresponding to the shapes of the core sections BA (the core sections BA being smaller).

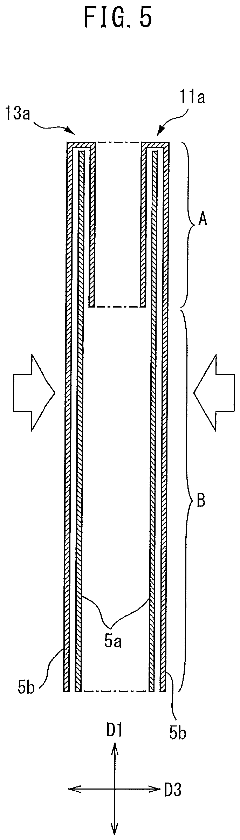

Fig. 5 is a schematic view showing an example of the construction of a cover sheet at the joint section 14a of Fig. 3. The drawing schematically shows an example of the laminated state of the cover sheet 5 at the joint section 14a, as seen from an end in the transverse direction D2. In this drawing, however, the elastic members 8a, 8b, 8c are omitted. The joint section 14b has the same end shape as the joint section 14a, and its explanation will therefore be omitted. For this embodiment, at the end section 11a of the abdomen side section 11, the end on the waist opening WO side in the longitudinal direction D1 of the cover sheet 5b is folded over to the skin side so as to cover the end in the longitudinal direction D1 of the cover sheet 5a. Thus, the end section 11a has region A where three layers of the cover sheets are layered, including the cover sheet 5b, the cover sheet 5a and the folded cover sheet 5b, and a region B where two layers of the cover sheets are layered, including the cover sheet 5a and the cover sheet 5b. Similarly, at the end section 13a of the dorsal side section 13, the end on the waist opening WO side in the longitudinal direction D1 of the cover sheet 5b is folded over to the skin side so as to cover the end in the longitudinal direction D1 of the cover sheet 5a. The end section 13a thus has a region A where three layers of the cover sheets are layered and a region B where two layers of the cover sheets are layered. Also, as indicated by the outline arrows in the drawing, the end section 11a and end section 13a are layered so as to overlap in the thickness direction D3, and are fused and joined at each of the fused sections by a fusing apparatus (described hereunder), thus constructing the joint section 14a. For this embodiment, the end section 11a and end section 13a join together the respective regions A and regions B. The size of region A is not particularly restricted and may be 10 mm to 100 mm, for example. There are no particular restrictions on the number of layers of the cover sheet 5 on the abdomen side section 11 and dorsal side section 13, and it may be a single layer or multiple layers, such as two or more layers. The cover sheet 5 also does not need to be folded.

In the disposable diaper 1 (absorbent article), the fused sections 21Db (to 23Db) and 21Ub (to 23Ub) each have a core section BA and a perimeter wall section BW (BW1-BW4) surrounding the core section BA in a tubular manner. That is, since the disposable diaper 1 has the perimeter wall sections BW, it is possible to stably prevent separation of the bonds of the joint sections 14a, 14b by movement of the wearer while the disposable diaper 1 is being worn. In the fused sections 21Db (to 23Db) and 21Ub (to 23Ub), in the transverse direction D2 where the difference in width of the perimeter wall section BW is large, the first dimension PI, which is the width of the first perimeter wall section BW1 in the transverse direction first side D2I, where the width is small, is equal to or more than 40% and less than 100% of the second dimension P2, which is the width of the second perimeter wall section BW2 in the transverse direction second side D2II, where the width is large. That is, it is formed so that the difference between the width of the first perimeter wall section BW1 on the transverse direction first side D2I and the width of the second perimeter wall section BW2 on the transverse direction second side D2II is not very large, but instead small. Therefore, the fused sections 21Db (to 23Db) and 21Ub (to 23Ub) also each include the third perimeter wall section BW3 and the fourth perimeter wall section BW4 with a small difference in the widths of the perimeter wall sections BW on both outer sides of the core section BA in the longitudinal direction D1, the widths of the perimeter wall sections BW being relatively uniform overall, without sections having widths that are excessively thinner than the other sections.

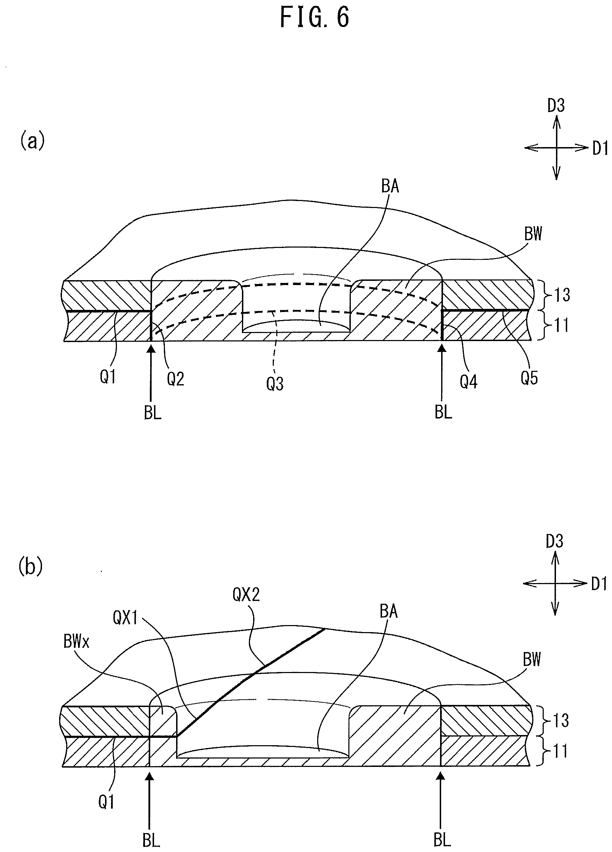

Fig. 6 is a schematic view illustrating the function of a disposable diaper according to an embodiment. Fig. 6(a) shows a fused section of the embodiment described above, and Fig. 6(b) shows a fused section including a perimeter wall section having section with an excessively thin width. In Fig. 6(b), a section BWx with an excessively thin width is present in the perimeter wall section BW. Therefore, when the abdomen side section 11 and the dorsal side section 13 are peeled apart, the peeling force first causes the abdomen side section 11 and dorsal side section 13 to tear on a break line Q1. When the tear reaches the section BWx of the perimeter wall section BW with excessively thin width, the weak section BWx of excessively thin width can potentially tear along the break line QX1. When this occurs, the tear propagates beyond the fused section to the sheet member on the dorsal side section 13 (or abdomen side section 11), such that the sheet member of the dorsal side section 13 may possibly tear along the break line QX2. In such a case, it becomes impossible to peel apart the abdomen side section 11 and dorsal side section 13, and the absorbent body becomes exposed or damaged between the gaps of the torn sheet members, potentially resulting in exposure or leakage of excreta.

In Fig. 6(a), on the other hand, i.e. according to this embodiment, no section of excessively thin width is present in the perimeter wall section BW. Consequently, when the abdomen side section 11 and dorsal side section 13 are peeled apart, the peeling force causes tearing between the abdomen side section 11 and dorsal side section 13 to first occur on the break line Q1. When the tear reaches the perimeter wall section BW, the tear propagates not along the thick, strong perimeter wall section BW, but rather on the border section BL between the sheet member and the perimeter wall section BW on the abdomen side section 11 (or dorsal side section 13) (the perimeter section of the fused section), which has relatively weak strength. In this case, the border section BL between the sheet member and the perimeter wall section BW tears along the break lines Q2, Q3 and Q4. The abdomen side section 11 and dorsal side section 13 then tear along the break line Q5.

Thus, with for this embodiment, the width of the perimeter wall section BW is relatively uniform overall, there being no sections with excessively thinner widths than the other sections, and therefore tearing of the perimeter wall section BW by peeling force can be minimized when the abdomen side section 11 and dorsal side section 13 are peeled apart. Furthermore, the border sections between the sheet members and perimeter wall sections BW, i.e. the perimeter sections of the fused sections, can be torn apart. As a result, the joint sections can be fully peeled apart into the abdomen side section and dorsal side section along the longitudinal direction, and the situation that the perimeter wall sections are torn, tearing proceeds from the torn sections and the sheet member is torn at sections other than the joint sections can be minimized. Thus, the absorbent article with joint sections makes it possible to stably prevent separation of the joint sections due to movement by the wearer when the absorbent article is being worn, and to allow the abdomen side section and dorsal side section to be properly and easily separated without tearing of the sheet members, when the absorbent article is removed. Incidentally from the viewpoint of uniformity of the width of the perimeter wall section BW that allows the function and effect to be exhibited, the first dimension P1 is preferably equal to or more than 50% and less than 100% of the second dimension P2, and more preferably it is equal to or more than 55% and less than 100% and even more preferably equal to or more than 60% and less than 100% of the second dimension P2. The first dimension P1 may be up to 90% of the second dimension P2 that allows the function and effect to be exhibited.

Incidentally, in the joint section 14a, it is not necessary for all of the plurality of fused sections 21Db to 23Db to each include the core section BA and perimeter wall section BW having the above-described technical features. The ratio of the number of the fused sections which each include the core section BA and perimeter wall section BW having the above-described technical features to the number of the plurality of fused sections 21Db to 23Db is equal to or more than 80 %, preferably equal to or more than 90 %, and more preferably equal to or more than 95 %. This is the same as the plurality of fused sections 21Ub to 23Ub in the joint section 14b.

Preferably, the fused sections which do not include the core section BA and perimeter wall section BW having the above-described technical features are not positioned in the end section of the waist opening WO side and/or the leg openings LO side where the wearer starts peeling apart the abdomen side section and the dorsal side section along the longitudinal direction D1 (first direction). Preferably, the fused sections which do not include the core section BA and perimeter wall section BW having the above-described technical features are not positioned adjacent to each other in the longitudinal direction D1 (first direction). These allows the abdomen side section and dorsal side section to be properly and easily separated without tearing of the sheet members, when the absorbent article is removed.

As a preferred mode of this embodiment, in a disposable diaper (absorbent article), the width of the third perimeter wall section BW3 or fourth perimeter wall section BW4 located on both outer sides of the core section BA in the longitudinal direction D1 (first direction) gradually thickens toward the transverse direction D2 (second direction). Therefore, the width varies in a continuous manner from the first perimeter wall section BW1 of small thickness on the transverse direction first side D2I in the transverse direction D2 (the first dimension P1), through the third perimeter wall section BW3 or fourth perimeter wall section BW4 located on both outer sides of the core section BA in the longitudinal direction D1, up to the second perimeter wall section BW2 of large thickness on the transverse direction second side D2II in the transverse direction D2 (the second dimension P2). That is, the fused sections 21Db (to 23Db) and 21Ub (to 23Ub) have gradually varying widths of their perimeter wall sections BW surrounding the core sections BA while being relatively uniform overall, and therefore do not have sections with excessively smaller thickness than the other sections. Thus, it is possible to more stably prevent separation of the bonds of the joint sections 14a, 14b, caused by movement of the wearer when the article is worn. Furthermore, when the abdomen side section 11 and the dorsal side section 13 are peeled apart, it is possible to further minimize tearing of the perimeter wall sections BW caused by the peeling force, so that tearing more readily occurs at the perimeter sections of the fused sections 21Db (to 23Db) and 21Ub (to 23Ub). The degree to which the widths of the perimeter wall sections BW increase from the transverse direction first side D2I toward transverse direction second side D2II may be such that the proportion of the minimum dimension P3min, P4min of the third perimeter wall section BW3 or fourth perimeter wall section BW4, is in the range of equal to or more than 60% and less than 100%, for example, of the maximum dimension. It is preferably equal to or more than 60%, since the uniformity of the perimeter wall section BW width will increase. The minimum dimension is more preferably in the range of equal to or more than 80% and less than 100% of the maximum dimension.

As a preferred mode of this embodiment, in the disposable diaper (absorbent article), the widths of the perimeter wall sections BW (BW3, BW4) at arbitrary locations in the transverse direction D2 (second direction) at the third perimeter wall section BW3 and fourth perimeter wall section BW4 located opposite each other on both outer sides of the core section BA, are the same within a range of ±15%, in the longitudinal direction D1 (first direction). In this case, the fused sections 21Db (to 23Db) and 21Ub (to 23Ub) have the perimeter wall section BW widths even more uniform overall, and therefore further do not have sections with excessively smaller thickness than the other sections. It is thus possible to further minimize tearing of the perimeter wall sections BW caused by peeling force, so that tearing more readily occurs at the perimeter sections of the fused sections 21Db (to 23Db) and 21Ub (to 23Ub).

In a disposable diaper (absorbent article) according to a preferred mode of this embodiment, the maximum dimension of the core section in the second direction is longer than the maximum dimension of the core section in the first direction, and therefore the fused sections have long horizontal shapes in the second direction. Therefore, compared to when the fused sections do not have horizontally long shapes, force applied to the joint sections by movement of the wearer when the article is worn can be more stably received, and separation of the joint sections can be more stably prevented. Furthermore, since the fused sections are relatively long in the second direction and hence the perimeter wall sections with small differences in width at both outer sides of the core section are longer in the second direction, it is possible to further minimize sections where the widths of the perimeter wall sections are too thin. Consequently, it is possible to further minimize tearing of the perimeter wall sections caused by peeling force when the abdomen side section and the dorsal side section are peeled apart, so that tearing more readily occurs at the perimeter sections of the fused sections. As the degree of horizontal length, preferably the maximum dimension d1 is 30 to 80% of the maximum dimension d2, for example. If it is equal to or more than 30%, the fusibility can be more stable, and if it is no greater than 80%, the ease of peeling apart will be increased.

In a disposable diaper (absorbent article) according to a preferred mode of this embodiment, the first dimension P1 in the transverse direction D2 of the perimeter wall section BW (on the side with the smaller width) is equal to or more than 0.5% of the maximum dimension d2 in the transverse direction D2 of the core section BA (which is defined as 100%). That is, the first dimension P1 has a width that is at least a prescribed width (0.5% of the maximum dimension d2 of the core section BA). Thus, the fused sections 21Db (to 23Db) and 21Ub (to 23Ub) do not have sections with excessively smaller thickness than the other sections in the perimeter wall section BW. Therefore, it is possible to further minimize tearing of the perimeter wall sections BW caused by peeling force, so that tearing more readily occurs at the perimeter sections of the fused sections 21Db (to 23Db) and 21Ub (to 23Ub). From the viewpoint of minimizing sections of excessively thin width in the perimeter wall section BW, the first dimension P1 is more preferably equal to or more than 1% and even more preferably at least 5% of the maximum dimension d2. From the viewpoint of easier peeling apart, the first dimension P1 is more preferably no greater than 50% and even more preferably no greater than 20% of the maximum dimension d2.

In a disposable diaper (absorbent article) according to a preferred mode of this embodiment, the first dimension P1 of the first perimeter wall section BW1 located on the transverse direction first side D2I (inner side) of the core section BA, at the joint section 14a on the transverse direction second side D2II, is equal to or more than 40% and less than 100% of the second dimension P2 of the second perimeter wall section BW2 located on the transverse direction second side D2II (inner side) of the core section BA, at the joint section 14b on the transverse direction first side D2I. Therefore, for a single fused section 21Db (to 23Db, 21Ub to 23Ub), the difference between the width of the first perimeter wall section BW1 on the transverse direction first side D2I and the width of the second perimeter wall section BW2 on the transverse direction second side D2II is small, while for a pair of joint sections 14a, 14b, the difference between the width of the first perimeter wall section BW1 on the transverse direction first side D2I at the joint section 14a on the transverse direction second side D2II and the width of the second perimeter wall section BW2 on the transverse direction second side D2II at the joint section 14b on the transverse direction first side D2I is also small. In other words, for the pair of joint sections 14a, 14b, the widths of the perimeter wall sections BW are relatively uniform overall at the fused sections 21Db (to 23Db) and 21Ub (to 23Ub), at all of the joint sections 14. Therefore, when the abdomen side section 11 and the dorsal side section 13 are peeled apart, it is possible to further minimize tearing of the perimeter wall sections BW caused by the peeling force, so that tearing more readily occurs at the perimeter sections of the fused sections 21Db (to 23Db) and 21Ub (to 23Ub). In this case, from the viewpoint of uniformity of the widths of the perimeter wall sections BW of the pair of joint sections 14a, 14b, the first dimension P1 of the joint section 14a is preferably equal to or more than 50% and less than 100% and more preferably equal to or more than 60% and less than 100% of the second dimension P2 of the joint section 14b.

In a disposable diaper (absorbent article) according to a preferred mode of this embodiment, at least the joint section 14a on the transverse direction second side D2II further includes a plurality of auxiliary fused sections 21Da (to 23Da) on the transverse direction first side D2I of the plurality of fused sections 21Db (to 23Db), the plurality of auxiliary fused sections 21Da (to 23Da) including an auxiliary core section and an auxiliary perimeter wall section wherein the area of the auxiliary core section is smaller than the area of the core section. Thus, compared to when the joint sections 14a do not have a plurality of auxiliary fused sections 21Da (to 23Da) on the transverse direction first side D2I, force applied to the joint sections by movement of the wearer when the article is worn can be more stably received, and separation of the joint section 14a can be more stably prevented.

In a disposable diaper (absorbent article) according to a preferred mode of this embodiment, both of the pair of joint sections 14a, 14b further include a plurality of auxiliary fused sections 21Da (to 23Da) and 21Ua (to 23Ua) on the transverse direction first side D2I of the plurality of fused sections 21Db (to 23Db) and 21Ub (to 23Ub). That is, at the joint section 14b on the transverse direction first side D2I, of the pair of joint sections 14a, 14b, the auxiliary fused sections 21Ua (to 23Ua) are present on the side receding from the longitudinal center line CL (imaginary line), i.e. on the outer side in the transverse direction D2, with respect to the fused sections 21Ub (to 23Ub). At the same time, at the joint section 14a on the transverse direction second side D2II, of the pair of joint sections 14a, 14b, the auxiliary fused sections 21Da (to 23Da) are present on the side approaching the longitudinal center line CL, i.e. the inner side in the transverse direction D2, with respect to the fused sections 21Db (to 23Db). At the pair of joint sections 14a, 14b, therefore, the fused sections 21Ub (to 23Ub) and 21Db (to 23Db) and the auxiliary fused sections 21Ua (to 23Ua) and 21Da (to 23Da) may be constructed asymmetrically with respect to the longitudinal center line CL. Therefore, by having the relatively small auxiliary fused sections 21Ua (to 23Ua) on the outer side in the transverse direction D2 at the joint section 14b on the transverse direction first side D2I, it is possible for the portion on the outer side in the transverse direction D2 to be easily deformed (easily rounded) inward. On the other hand, by having the relatively large fused sections 21Db (to 23Db) on the outer side in the transverse direction D2 at the joint section 14a on the transverse direction second side D2II, it is possible for the portion on the outer side in the transverse direction D2 to be less easily deformed (less easily rounded) inward. As a result, at the pair of joint sections 14a, 14b, it is possible to produce right-left asymmetry for not only the arrangement of the fused sections 21Ub (to 23Ub) and 21Db (to 23Db) and the auxiliary fused sections 21Ua (to 23Ua) and 21Da (to 23Da), but also their overall shapes (degree of rounding). Therefore, by visual and/or tactile confirmation of the arrangement of the fused sections 21Ub (to 23Ub) and 21Db (to 23Db) and the auxiliary fused sections 21Ua (to 23Ua) and 21Da (to 23Da) and the overall asymmetry shapes of the joint sections 14a, 14b, it is possible to easily distinguish left and right in the transverse direction D2 of the absorbent article. As a result, the thickness direction D3 regions, i.e. the abdomen side section 11 and dorsal side section 13 of the absorbent article, can be easily recognized and distinguished (when the right/left relationships of the arrangement or shapes has been learned in advance from the package of the absorbent article (such as a disposable diaper)).

In a disposable diaper (absorbent article) according to a preferred mode of this embodiment, the shapes of the core sections of the auxiliary fused sections 21Ua (to 23Ua) and 21Da (to 23Da) are circular. Thus, the shapes of the auxiliary fused sections 21Ua (to 23Ua) and 21Da (to 23Da) including the perimeter wall sections are also circular. Therefore, force from different directions, applied to the joint sections 14a, 14b by movement of the wearer when the article is worn can be more stably received, and separation of the bonds of the joint sections 14a, 14b can be more stably prevented.

In a disposable diaper (absorbent article) according to a preferred mode of this embodiment, the plurality of fused sections 21Ub (to 23Ub) and 21Db (to 23Db) of the pair of joint sections 14a, 14b are disposed along oblique directions with respect to the longitudinal direction D1, so as to gradually recede from the waist side toward the leg side or from the leg side toward the waist side, with respect to the longitudinal center line CL (imaginary line). That is, the plurality of fused sections 21Ub (to 23Ub) and 21Db (to 23Db) of the pair of joint sections 14a, 14b are disposed in a divergent shape from the waist side toward the leg side or from the leg side toward the waist side. Therefore, in the transverse direction of the waist side or leg side, protrusion sections (ears) 14aT1, 14bT1 that protrude toward the outer sides can be formed at the portions further on the outer sides than the plurality of fused sections 21Ub (to 23Ub) and 21Db (to 23Db) at the joint sections 14a, 14b (the portions further on the outer sides than the fused sections 21Ub (to 23Ub) and 21Db (to 23Db) with respect to the longitudinal center line CL). Thus, when the wearer removes the absorbent article, the protrusion sections 14aT1, 14bT1 are gripped and pulled to the outer side in the transverse direction D2, thereby allowing a gap to be formed between the portion on the waist side or leg side of the absorbent article and the body, to facilitate insertion of the finger. Alternatively, it may facilitate insertion of a finger at the section between the body and the portions 14aT2, 14bT2 of the leg side or waist side opposite the protrusion sections 14aT1, 14bT1 in the longitudinal direction D1 of the absorbent article. A finger can thus be inserted into the gaps, allowing the abdomen side section 11 and dorsal side section 13 to be easily peeled apart.

Incidentally, the pair of joint sections 14a, 14b may further include different fused sections with different constructions from the fused sections shown in Fig. 4, so long as they do not cause tearing to propagate in the sheet member as shown in Fig. 6(b), when the abdomen side section 11 and dorsal side section 13 are peeled apart.

An example of a method for producing a disposable diaper 1 according to an embodiment of the present invention will now be described.

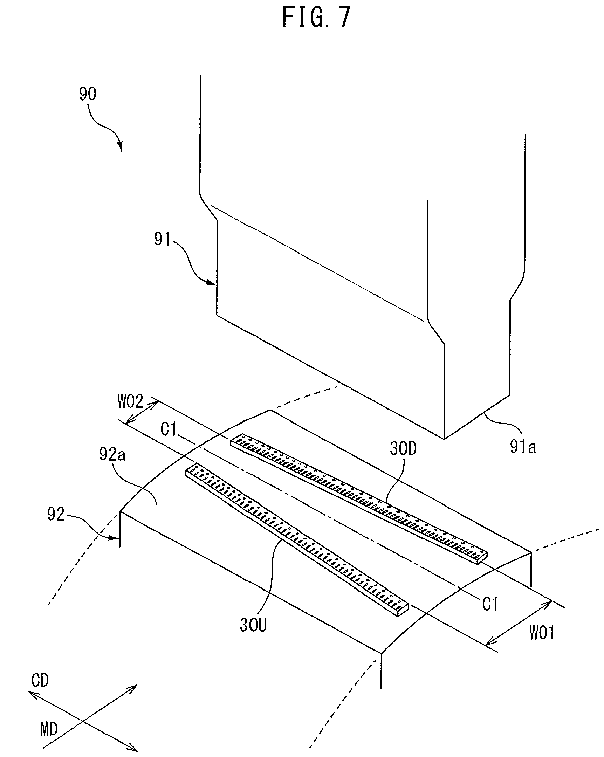

First, an example of a fusing apparatus for formation of joint sections will be explained, which is to be used in the present production method. Fig. 7 is a schematic view showing an example of the construction of a fusing apparatus according to an embodiment. The fusing apparatus 90 includes a rotor (not shown) provided with an anvil 92 (first clamping member) having a seal-facing surface 92a, and an ultrasonic horn 91 (second clamping member) having a seal-facing surface 91a. The anvil 92 is disposed inside the rotor in such a manner that the seal-facing surface 92a is approximately flush with the outer peripheral surface of the rotor. The seal-facing surface 92a includes a downstream protrusion row 30D and an upstream protrusion row 30U, in each of which a plurality of protrusions are arranged in a row for formation of the fused sections. The downstream protrusion row 30D and upstream protrusion row 30U are located downstream and upstream, respectively, in the machine direction MD of the absorbent article semi-product during production. The plurality of protrusions of the downstream protrusion row 30D and upstream protrusion row 30U extend along the cross-machine direction CD that is perpendicular to the machine direction MD. The seal-facing surface 91a has an area facing either or both the downstream protrusion row 30D and upstream protrusion row 30U.

The absorbent article semi-product is transported in the machine direction MD between the anvil 92 and ultrasonic horn 91, along the outer peripheral surface of the rotor. The anvil 92 and ultrasonic horn 91 are able to approach each other in the height direction TD that is approximately perpendicular to the machine direction MD. However, the ultrasonic horn 91 undergoes reciprocal movement in a prescribed range in a direction along the outer peripheral surface of the rotor that includes the anvil 92 (the machine direction MD). The prescribed range is a range of a prescribed peripheral length (arc) along the outer peripheral surface, and it may be a range of within 30° as the corresponding rotational angle of the rotor, for example, or a range of within 5 times the distance between the downstream end of the downstream protrusion row 30D and the upstream end of the upstream protrusion row 30U, for example. Thus, the ultrasonic horn 91 moves from the upstream end to the downstream end in the circumferential direction (machine direction MD) (following the outer peripheral surface) within a prescribed range, while clamping the semi-product with the anvil 92, after which it separates from the semi-product and moves from the downstream end to the upstream end. When the ultrasonic horn 91 moves from the upstream end to the downstream end, the downstream protrusion row 30D on the seal-facing surface 92a of the anvil 92 and the seal-facing surface 91a of the ultrasonic horn 91 approach each other in the height direction TD and clamp the abdomen side section and dorsal side section of the absorbent article semi-product, thus producing fusion by ultrasonic vibration (and pressure) to form the joining rows 20D. Together with that, the upstream protrusion row 30U on the seal-facing surface 92a and the seal-facing surface 91a clamp the abdomen side section and dorsal side section of the semi-product, thus producing fusion by ultrasonic vibration (and pressure) to form the joining row 20U. At this time, the joining row 20D of the semi-product at the downstream end (at right in Fig. 3) and the joining row 20U of the semi-product at the upstream end (at left in Fig. 3) are formed, of the two semi-products aligned with the machine direction MD parallel to the transverse direction D2. The joining row 20D and joining row 20U may be formed simultaneously, or the joining row 20D may be formed first and the joining row 20U formed afterwards. The apparatus of Patent Literature 1 may be mentioned as an example of the fusing apparatus 90. Incidentally, the ultrasonic horn 91 does not need to have reciprocal movement in the machine direction MD.

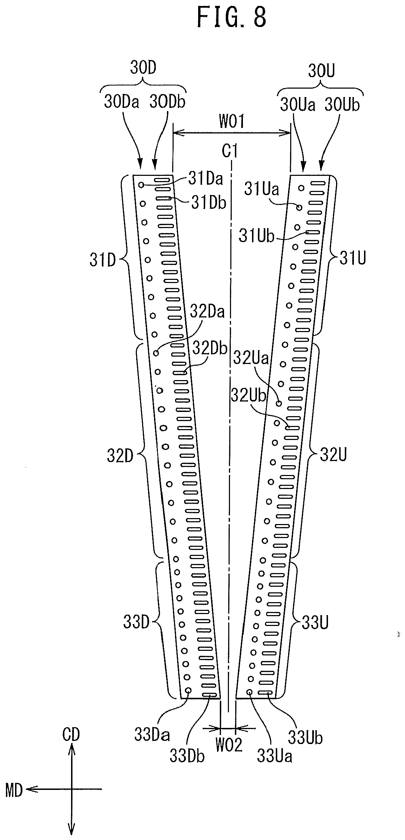

Fig. 8 is a schematic view showing an example of the construction of protrusion rows on an anvil 92 of a fusing apparatus 90. The downstream protrusion row 30D and upstream protrusion row 30U each have, in the cross-machine direction CD, waist side regions 31D, 31U, leg side regions 33D, 33U, and center regions 32D, 32U between the waist side regions 31D, 31U and leg side regions 33D, 33U. The waist side regions 31D, 31U correspond to the waist side regions 21D, 21U of the joint sections 14a, 14b, respectively. The leg side regions 33D, 33U correspond to the leg side regions 23D, 23U of the joint sections 14a, 14b, respectively. The center regions 32D, 32U correspond to the center regions 22D, 22U of the joint sections 14a, 14b, respectively.

For this embodiment, the downstream protrusion row 30D includes a temporary fastening protrusion row 30Da located downstream in the machine direction MD (left in Fig. 8) and extending in the cross-machine direction CD, and a main fastening protrusion row 30Db located upstream in the machine direction MD (right side in Fig. 8) and extending in the cross-machine direction CD. The temporary fastening protrusion row 30Da includes a plurality of temporary fastening protrusions 31Da, 32Da, 33Da, each aligned in the cross-machine direction CD, in the waist side region 31D, center region 32D and leg side region 33D. Also, the main fastening protrusion row 30Db includes a plurality of main fastening protrusions 31Db, 32Db, 33Db, each aligned in the cross-machine direction CD, in the waist side region 31D, center region 32D and leg side region 33D. The plurality of temporary fastening protrusions 31Da, 32Da, 33Da are used to form the plurality of auxiliary fused sections 21Da, 22Da, 23Da. The plurality of main fastening protrusions 31Db, 32Db, 33Db are used to form the plurality of fused sections 21Db, 22Db, 23Db.

Also for this embodiment, the upstream protrusion row 30U includes a temporary fastening protrusion row 30Ua located downstream in the machine direction MD (left in Fig. 7) and extending in the cross-machine direction CD, and a main fastening protrusion row 30Ub located upstream in the machine direction MD (right side in Fig. 7) and extending in the cross-machine direction CD. The temporary fastening protrusion row 30Ua includes a plurality of temporary fastening protrusions 31Ua, 32Ua, 33Ua, each aligned in the cross-machine direction CD, in the waist side region 31U, center region 32U and leg side region 33U. Also, the main fastening protrusion row 30Ub includes a plurality of main fastening protrusions 31Ub, 32Ub, 33Ub, each aligned in the cross-machine direction CD, in the waist side region 31U, center region 32U and leg side region 33U. The plurality of temporary fastening protrusions 31Ua, 32Ua, 33Ua are used to form the plurality of auxiliary fused sections 21Ua, 22Ua, 23Ua. The plurality of main fastening protrusions 31Ub, 32Ub, 33Ub are used to form the plurality of fused sections 21Ub, 22Ub, 23Ub.

Each of the plurality of temporary fastening protrusions 31Da to 33Da have smaller areas than each of the main fastening protrusions 31Db to 33Db, and therefore have shorter outer peripheral lengths. Specifically, the temporary fastening protrusions 31Da have smaller areas than the main fastening protrusions 31Db, preferably the temporary fastening protrusions 32Da have smaller areas than the main fastening protrusions 32Db, and preferably the temporary fastening protrusions 33Da have smaller areas than the main fastening protrusions 33Db. The temporary fastening protrusions 33Da also preferably have smaller areas than the main fastening protrusions 31Db. These relationships also apply to the outer peripheral lengths. When the shapes are circular, the sizes of each of the temporary fastening protrusions 31Da to 33Da may be 0.5 to 5 mm diameters, for example. When the shapes are long oblong (rounded rectangular) in the transverse direction D2, the sizes of each of the main fastening protrusions 31Db to 33Db may have short sides (short diameters) of 0.5 to 5 mm and long sides (long diameters) of 1 to 20 mm, for example. The distances between the adjacent temporary fastening protrusion row 30Da and main fastening protrusion row 30Db may be 1 to 10 mm, for example, the distance between the adjacent temporary fastening protrusions may be 1 to 30 mm, for example, and the distance between adjacent main fastening protrusions may be 1 to 10 mm, for example. The intervals between adjacent protrusions may be constant or varying based on location. The intervals between the temporary fastening protrusions and main fastening protrusions may be 1 to 30 mm, for example.

For this embodiment, the construction of each of the plurality of temporary fastening protrusions 31Ua to 33Ua and each of the plurality of main fastening protrusions 31Ub to 33Ub are the same as the construction of each of the plurality of temporary fastening protrusions 31Da to 33Da and each of the plurality of main fastening protrusions 31Db to 33Db.

The overall arrangement of the downstream protrusion row 30D and upstream protrusion row 30U is symmetrical with respect to a center line C1 running in the cross-machine direction CD through the center of the seal-facing surface 92a in the machine direction MD. Considering the arrangement of the protrusions, however, the downstream protrusion row 30D and upstream protrusion row 30U are asymmetrical with respect to the center line C1. Therefore, the temporary fastening protrusion row 30Da and main fastening protrusion row 30Db and the temporary fastening protrusion row 30Ua and main fastening protrusion row 30Ub are asymmetrical with respect to the center line C1. Thus, the plurality of temporary fastening protrusions 31Da to 33Da and the plurality of main fastening protrusions 31Db to 33Db, and the plurality of temporary fastening protrusions 31Ua to 33Ua and the plurality of main fastening protrusions 31Ub to 33Ub, are asymmetrical with respect to the center line C1.

For this embodiment, the distance between the downstream protrusion row 30D and upstream protrusion row 30U is W01 at the end of the waist side regions 31D, 31U, and W02 at the end of the leg side regions 33D, 33U, the relationship between them being W01 ≥ W02. Thus, the downstream protrusion row 30D and upstream protrusion row 30U are formed in an approximate V-shape (divergent shape), as seen from the height direction that is perpendicular to the machine direction MD and cross-machine direction CD. The angle formed between them is preferably 0 to 20 degrees, for example, and more preferably 2 to 10 degrees from the viewpoint of stable formation of the fused sections. However, if the angle formed between them is greater than 20°, the proportion of the pair of joint sections 14a, 14b occupying the disposable diaper 1 will increase, resulting in excessive material usage. In addition, if the angle formed between them is greater than 20°, the width near the waist opening WO of the disposable diaper 1 will significantly differ from the width near the upper side of the leg opening LO, making it difficult to match the body frame of the wearer.

For this embodiment, the downstream protrusion row 30D and upstream protrusion row 30U in each region both consist of two rows. However, the downstream protrusion row 30D may consist of more than two rows, and the number of rows may differ for each region. The upstream protrusion row 30U, likewise, may consist of one row or more than two rows, and the number of rows may differ for each region.

For this embodiment, of the downstream protrusion row 30D (, upstream protrusion row 30U), the temporary fastening protrusion row 30Da (, 30Ua) is disposed in the downstream section in the machine direction MD, while the main fastening protrusion row 30Db (, 30Ub) is disposed in the upstream section. As a result, the temporary fastening protrusion row 30Da clamps the absorbent article semi-product first during production, and the main fastening protrusion row 30Db clamps it afterwards. Thus, it is possible to first carry out the temporary fastening fusion and then the main fastening fusion, at the abdomen side section and dorsal side section ends of the semi-product. If the machine direction MD is reversed, the downstream protrusion row 30D and upstream protrusion row 30U in Fig. 7 will be reversed on the right and left sides with respect to the center line C1. Correspondingly, the joining row 20D and joining row 20U in Fig. 3 will be reversed on the right and left sides with respect to the longitudinal center line CL.

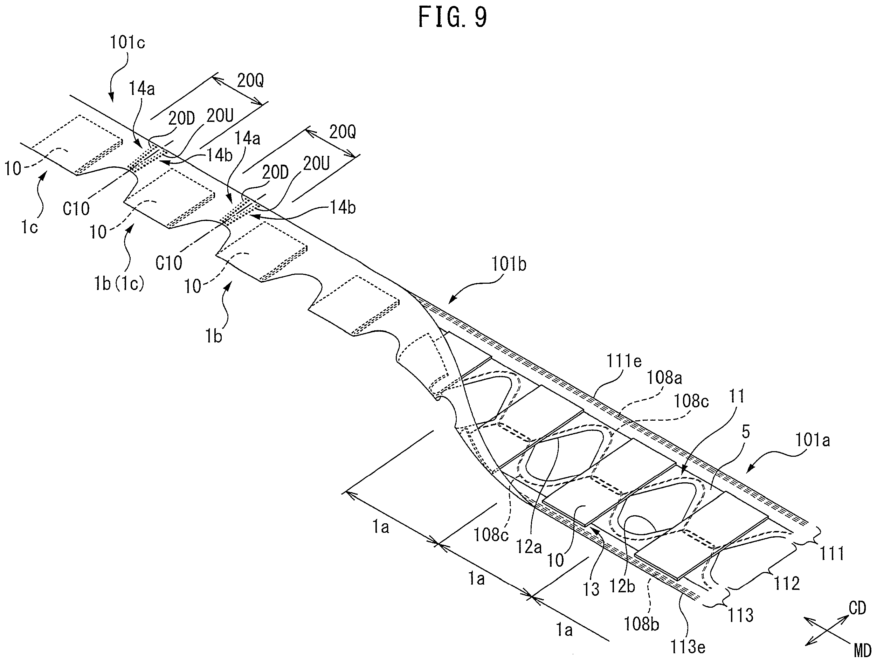

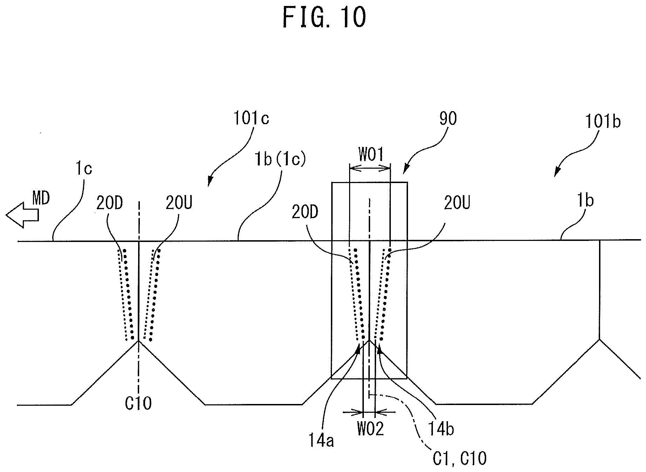

An example of a method for producing a disposable diaper 1 according to an embodiment of the present invention will now be described. Fig. 9 is a perspective view showing a method for producing a disposable diaper 1 according to an embodiment, the overlaying step and joining step of the production method being illustrated. Also, Fig. 10 is a schematic view of the joining step of the production method of Fig. 9, i.e. the process of forming the joint sections 14a, 14b. The production method includes an overlaying step and a forming step. These will now be explained in detail.

First, a continuous sheet member 101a is formed by a conventionally known production method. The continuous sheet member 101a includes an abdomen-side continuous section 111 having a plurality of the abdomen side sections 11 connected in the transverse direction D2 (machine direction MD), and a dorsal-side continuous section 113 having a plurality of the dorsal side sections 13 connected in the transverse direction D2 (machine direction MD), on either side in the longitudinal direction D1 (cross-machine direction CD). Incidentally, the continuous sheet member 101a also includes a middle continuous section 112 having a plurality of the crotch sections 12 connected in the transverse direction D2 (machine direction MD), between the abdomen-side continuous section 111 and dorsal-side continuous section 113. In the continuous sheet member 101a, the absorbent body 10 is disposed on the abdomen side section 11, crotch section 12 and dorsal side section 13 aligned in the longitudinal direction D1 (cross-machine direction CD), and collectively they may be considered to be the semi-product 1a of the disposable diaper 1 (Fig. 2). That is, the continuous sheet member 101a can be considered to have a structure in which the semi-products 1a of the disposable diapers 1 (Fig. 2) in the expanded state are connected in the transverse direction D2 (machine direction MD). One edge section 111e of the continuous sheet member 101a in the cross-machine direction CD includes an elastic member 108a for the elastic member 8a, in the continuous sheet member 101a, while the other edge section 113e includes an elastic member 108b for the elastic member 8b. Elastic members 108c, 108c for the elastic members 8c, 8c are also disposed in the continuous sheet member 101a. The elastic members 108c, 108c both extend in the machine direction MD while curving in an undulating fashion. The side sections 12a, 12b that are to be the leg openings LO of the disposable diaper 1 are formed in the regions surrounded by the elastic members 108c, 108c.

An overlaying step is then carried out, in which the abdomen-side continuous section 111 and dorsal-side continuous section 113 are laid one over the other. Specifically, in the overlaying step, the continuous sheet member 101a is transported in the machine direction MD, with the transverse direction D2 along the machine direction MD and the longitudinal direction D1 along the cross-machine direction CD, while the dorsal-side continuous section 113 is laid over the abdomen-side continuous section 111, and the abdomen side section 11 and dorsal side section 13 are overlaid. In other words, the dorsal-side continuous section 113 is folded onto the abdomen-side continuous section 111 so that the edge section 111e and edge section 113e overlap in the cross-machine direction CD. Thus, an absorbent article semi-product 1b is continuously formed having the abdomen side section 11 and dorsal side section 13 overlaid. That is, a continuous sheet member 101b is formed having absorbent article semi-products 1b continuously running in the machine direction MD. A publicly known folding apparatus may be used for folding (layering) of the dorsal-side continuous section 113 on the abdomen-side continuous section 111. The abdomen-side continuous section 111 may also be laid over the dorsal-side continuous section 113 in the overlaying step.

A joining step is carried out next, in which the pair of joint sections 14a, 14b are formed in the region 20Q straddling the two semi-products 1b, 1b that are adjacent in the machine direction MD. In the joining step, first the continuous sheet member 101b is supplied to the fusing apparatus 90 and is transported along the outer peripheral surface of a rotor. At the upstream end of the semi-product 1b at the downstream end, of two semi-products 1b, 1b that are adjacent in the machine direction MD, the abdomen side section 11 and dorsal side section 13 are clamped and fused during an extremely short period of time by the downstream protrusion row 30D of the anvil 92 (first clamping member), and the ultrasonic horn 91 (second clamping member), forming joint sections 14a. That is, in the joining step, first the joining rows 20D, i.e. the plurality of auxiliary fused sections 21Da, 22Da, 23Da, are formed in order at the temporary fastening protrusion row 30Da, and after a slight delay the plurality of fused sections 21Db, 22Db, 23Db are formed in order at the main fastening protrusion row 30Db. Following this, in the joining step, at the downstream end of the semi-product 1b at the upstream end, of two semi-products 1b, 1b that are adjacent in the machine direction MD, the abdomen side section 11 and dorsal side section 13 are clamped and fused during an extremely short period of time by the upstream protrusion row 30U of the anvil 92 (first clamping member), and the ultrasonic horn 91 (second clamping member), forming joint sections 14b. That is, in the joining step, the joining rows 20U, i.e. the plurality of auxiliary fused sections 21Ua, 22Ua, 23Ua, are formed in order at the temporary fastening protrusion row 30Ua, and after a slight delay the plurality of fused sections 21Ub, 22Ub, 23Ub are formed in order at the main fastening protrusion row 30Ub. These result in formation of the pair of joint sections 14a, 14b straddling two adjacent semi-products 1b, 1b. By continuously carrying out the joining step, a continuous sheet member 101c is formed having absorbent article semi-products 1c including the pairs of joint sections 14a, 14b, continuously connected in the machine direction MD. During this time, the continuous sheet member 101b is transported along the outer peripheral surface of the rotor at a prescribed speed in the machine direction MD (for example, 100 m/min), and with the ultrasonic horn 91 following along the outer peripheral surface of the rotor within a prescribed range (for example, within a 25° rotation angle of the rotor), the ultrasonic horn 91 is pressed against the continuous sheet member 101b in the height direction TD that is perpendicular to the machine direction MD. Thus, contact between the ultrasonic horn 91 and continuous sheet member 101b is only for a very short period of time, from about 10 ms to 100 ms, during which time they are clamped and fused.

A pair of transport rolls (not shown) are provided facing each other, at the upstream and downstream ends in the machine direction MD from the continuous sheet member 101a, 101b, 101c, as shown in Fig. 8. In the overlaying step and joining step, the continuous sheet member 101a, 101b, 101c is pulled at a prescribed tension in the machine direction MD between the pair of transport rolls at the upstream and downstream ends, while the continuous sheet member 101a is processed into the continuous sheet member 101c through the continuous sheet member 101b.

Next, in the continuous sheet member 101c, each downstream semi-product 1c is cut along the border line C10 between two adjacent semi-products 1c, 1c. The border line C10 corresponds to a line running through the center between the joining row 20D and joining row 20U in the machine direction MD, and extending in the cross-machine direction CD. A disposable diaper 1 is thus produced.

When the overlaying step is carried out for overlaying of the abdomen-side continuous section and dorsal-side continuous section, just before the forming step in which the continuous sheet member is transported in the machine direction while being clamped and sealed by the clamping member, the width of the perimeter wall section formed at the front end section of each protrusion (the downstream section in the machine direction), of the fused sections formed in the downstream protrusion rows and upstream protrusion rows, tends to become thinner, while the width of the perimeter wall section formed at the rear end section of each protrusion (the upstream section in the machine direction) tends to become thicker. The reason for this is as follows. When the dorsal-side continuous section is layered with the abdomen-side continuous section in the overlaying step, the dorsal-side continuous section temporarily moves obliquely with respect to the machine direction, and moves a longer distance in the machine direction than the abdomen-side continuous section. In this case, immediately after the overlaying step, the dorsal-side continuous section is in a more stretched state than the abdomen-side continuous section, and is therefore in a state of high tension. When the forming step is initiated while in this state, and the continuous sheet member is fused in contact with the front end sections (the sections at the downstream end) of the protrusions of the downstream protrusion rows, the highly-stretched dorsal-side continuous section and the not-highly-stretched abdomen-side continuous section become fused. When the fusing time is short, therefore, the fused sections (perimeter wall sections) cannot be adequately formed, and the widths of the perimeter wall sections formed by the front end sections of the protrusions (the sections at the downstream end) tend to become thinner. On the other hand, when the continuous sheet member that has contacted the rear end sections of the protrusions (the sections on the upstream end) are fused, since the fused sections (perimeter wall sections) formed by the front end sections of the protrusions (the sections on the downstream end) are already present in the continuous sheet member, the difference in tension between the dorsal-side continuous section and abdomen-side continuous section gradually decreases. Even if the fusing time is short, therefore, the fused sections (perimeter wall sections) can be adequately formed, and the widths of the perimeter wall sections formed by the rear end sections of the protrusions (the sections at the upstream end) tend to become thicker. As a result, it is difficult for the widths of the perimeter wall sections that are formed to be made basically uniform without depending on the locations surrounding the core sections, and the widths of the perimeter wall sections can potentially become partially thinned.

With this production method, however, it is possible to avoid a large difference in width between the perimeter wall sections of the fused sections formed by the front end sections (sections at the downstream end) of the (main fastening) protrusions and the perimeter wall sections of the fused sections formed by the rear end sections (the sections at the upstream end), or in other words, to avoid partial formation of excessively thin sections. Specifically, in the present production method, first the abdomen side section 11 and dorsal side section 13 of the continuous sheet member 101b are temporarily fastened at the auxiliary fused sections 21Da (to 23Da), on the upstream end of the semi-product 1b at the downstream end in the machine direction MD. Thus, the tension origins may be the auxiliary fused sections 21Da (to 23Da), i.e. they may be located further downstream than the locations in the overlaying step for the abdomen-side continuous section 111 and dorsal-side continuous section 113. It is thereby possible to minimize the difference between tension on the abdomen side section 11 and tension on the dorsal side section 13 of the continuous sheet member 101b, further upstream than the auxiliary fused sections 21Da (to 23Da). Next in the production method, main fastening of the abdomen side section 11 and dorsal side section 13 of the continuous sheet member 101b is carried out at the fused sections 21Db (to 23Db). Since main fastening can be carried out at this time while minimizing the difference in tension between the abdomen side section 11 and dorsal side section 13, it is possible to minimize the difference between the widths of the perimeter wall sections BW at the downstream section in the machine direction MD and the widths of the perimeter wall sections BW at the upstream section in the machine direction MD, for the fused sections 21Db (to 23Db), compared to when the auxiliary fused sections 21Da (to 23Da) are not provided.

Similarly, the abdomen side section 11 and dorsal side section 13 of the continuous sheet member 101b are temporarily fastened at auxiliary fused sections 21Ua (to 23Ua), on the downstream end of the semi-product 1b at the upstream end in the machine direction MD. Thus, the tension origins may be the auxiliary fused sections 21Ua (to 23Ua), i.e. they may be located further downstream than the locations in the overlaying step for the abdomen-side continuous section 111 and dorsal-side continuous section 113. It is thereby possible to minimize the difference between tension on the abdomen side section 11 and tension on the dorsal side section 13 of the continuous sheet member 101b, further upstream than the auxiliary fused sections 21Ua (to 23Ua). Next in the production method, main fastening of the abdomen side section 11 and dorsal side section 13 of the continuous sheet member 101b is carried out at the fused sections 21Ub (to 23Ub). Since main fastening can be carried out at this time while minimizing the difference in tension between the abdomen side section 11 and dorsal side section 13, it is possible to minimize the difference between the widths of the perimeter wall sections BW at the downstream section in the machine direction MD and the widths of the perimeter wall sections BW at the upstream section in the machine direction MD, for the fused sections 21Ub (to 23Ub), compared to when the auxiliary fused sections 21Ua (to 23Ua) are not provided.

In the method for producing a disposable diaper (absorbent article) according to a preferred mode of this embodiment, first the continuous sheet member 101b, in which absorbent article semi-products 1b where the abdomen side section 11 and dorsal side section 13 are layered together but not joined are connected in the transverse direction D2, is transported with the transverse direction D2 as the machine direction MD. Also, at least a plurality of fused sections 21Db (to 23Db) are each formed in order with a time difference at a location corresponding to one (14a) of the pair of joint sections 14a, 14b of the semi-product 1b (first forming step; first stage of the joining step). Next, after the first forming step, at least a plurality of fused sections 21Ub (to 23Ub) are each formed in order with a time difference at a location corresponding to the other (14b) of the pair of joint sections 14a, 14b of the semi-product 1b (second forming step; second stage of the joining step). It is thereby possible to reduce the number of fused sections formed per unit time, which are gripped and formed by the ultrasonic horn 91 and anvil 92 that form the fused sections 21Db (to 23Db) and 21Ub (to 23Ub). As a result, the gripping pressure (linear pressure) applied to each fused section can be increased. This allows the fused sections 21Db (to 23Db) and 21Ub (to 23Ub) to be stably formed, and can produce more uniform and adequate widths for the perimeter wall sections BW. Consequently, it is possible to further minimize tearing of the perimeter wall sections caused by peeling force when the abdomen side section and the dorsal side section are peeled apart, so that tearing more readily occurs at the perimeter sections of the fused sections.

In the example of the production method described above, in the joining step, first the plurality of auxiliary fused sections 21Da, 22Da, 23Da, are formed in order at the temporary fastening protrusion row 30Da, and after a slight delay the plurality of fused sections 21Db, 22Db, 23Db are formed in order at the main fastening protrusion row 30Db. Next, the plurality of auxiliary fused sections 21Ua, 22Ua, 23Ua, are formed in order at the temporary fastening protrusion row 30Ua, and after a slight delay the plurality of fused sections 21Ub, 22Ub, 23Ub are formed in order at the main fastening protrusion row 30Ub. However, the plurality of auxiliary fused sections 21Ua, 22Ua, 23Ua do not need to be formed at the temporary fastening protrusion row 30Ua. The reason for this is that forming the plurality of fused sections 21Db, 22Db, 23Db first can somewhat minimize the difference between the tension on the abdomen side section 11 and the tension on the dorsal side section 13 of the continuous sheet member 101b, due to the previously formed fused sections.

In the example of the production method described above, the plurality of auxiliary fused sections 21Da, 22Da, 23Da are formed before the plurality of fused sections 21Db, 22Db, 23Db, and the plurality of auxiliary fused sections 21Ua, 22Ua, 23Ua are formed before the plurality of fused sections 21Ub, 22Ub, 23Ub. Thus, the widths of the perimeter wall sections of the fused sections are relatively uniform. However, the method for producing a disposable diaper 1 is not limited to this method, and another method, such as a method that does not use temporary fastening protrusions (auxiliary fused sections) may be employed. For example, a method may be employed in which the energy supplied to the ultrasonic horn when the front end section of each main fastening protrusion (the downstream section) presses the sealing member is relatively high, while the energy supplied to the ultrasonic horn when the rear end section (the upstream section) presses the sealing member is relatively low. Alternatively, a method may be employed in which the energy of the ultrasonic horn supplied to the front end section of each main fastening protrusion (the downstream section) is relatively high, while the energy of the ultrasonic horn supplied to the rear end section (the upstream section) is relatively low. Still alternatively, the method may be one in which the heights of the front end sections (the downstream sections) of the main fastening protrusions from the outer peripheral surface of the anvil 92 are relatively high, while the heights of the rear end sections (the upstream sections) are relatively low. Yet alternatively, the method may be one in which the widths of the front end sections (the downstream sections) of the main fastening protrusions in the cross-machine direction CD are relatively wide, while the widths of the rear end sections (the upstream sections) are relatively narrow. These methods can also minimize the difference between the widths of the perimeter wall sections BW at the downstream section in the machine direction MD and the widths of the perimeter wall sections BW at the upstream section in the machine direction MD

As a preferred mode for this embodiment, the shapes of the plurality of fused sections 21Db (to 23Db) and/or 21Ub (to 23Ub) have longer lengths in the machine direction MD than their lengths in the cross-machine direction CD. That is, in the downstream protrusion row 30D and upstream protrusion row 30U, the shapes of the protrusions for the fused sections 21Db (to 23Db) and/or 21Ub (to 23Ub) (the main fastening protrusions 31Db (to 33Db) and/or 31Ub (to 33Ub)) are longitudinal in the machine direction MD. In the joining step, therefore, the contact time of the protrusions for the fused sections 21Db (to 23Db) and/or 21Ub (to 23Ub) with the continuous sheet member 101b may be longer, and therefore the time for formation of the fused sections 21Db (to 23Db) and/or 21Ub (to 23Ub) may be longer. It is thus possible to minimize the difference between the widths of the perimeter wall sections BW at the downstream section and the widths of the perimeter wall sections BW at the upstream section, for the fused sections 21Db (to 23Db) and/or 21Ub (to 23Ub).

As a preferred mode for this embodiment, in the downstream protrusion row 30D and upstream protrusion row 30U, the distance W02 between the ends on one side in the cross-machine direction CD is shorter than the distance W01 between the ends on the other side. That is, according to this preferred mode, the downstream protrusion row 30D and upstream protrusion row 30U of the anvil 92 (first clamping member) are formed in an approximate V-shape (divergent shape), as seen from the height direction that is perpendicular to the machine direction MD and cross-machine direction CD. During the joining step, therefore, the continuous sheet member 101b can be constantly pressed in the cross-machine direction CD by the anvil 92 (first clamping member) and ultrasonic horn 91 (second clamping member), so that the continuous sheet member 101b is less likely to be shifted in the cross-machine direction CD and the pair of joint sections 14a, 14b can be stably formed. It is therefore possible to minimize the difference between the widths of the perimeter wall sections BW at the downstream section and the widths of the perimeter wall sections BW at the upstream section, for the fused sections 21Db (to 23Db), and the widths of the perimeter wall sections BW at the downstream section and the perimeter wall sections BW at the upstream section, for the other fused sections 21Ub (to 23Ub). This can produce a better balance for the engaging strength of the pair of joint sections 14a, 14b, and allow them both to be peeled apart with approximately the same force. The angle formed between the downstream protrusion row 30D and upstream protrusion row 30U may be 0 to 20 degrees, for example, and it is preferably 2 to 10 degrees from the viewpoint of stable formation of the fused sections. If the angle formed between them is greater than 20 degrees, the proportion of the pair of joint sections 14a, 14b occupying the disposable diaper 1 will increase, resulting in excessive material usage during production. In addition, if the angle formed between them is greater than 20 degrees, the width near the waist opening WO of the disposable diaper 1 will significantly differ from the width near the upper side of the leg opening LO, making it difficult to match the body frame of the wearer.

### EXAMPLES

The present invention will now be explained using examples and comparative examples with reference to Fig. 3 and Fig. 4. However, the present invention is not limited only to the examples.

### (1) Sample

A disposable diaper described in the above embodiment was produced using the production method described in the above embodiment, as a disposable diaper for the Examples. A joint section 14a of a disposable diaper produced using an anvil 92 having temporary fastening protrusions as shown in Fig. 8 was cut out and used as a sample for Example 1, and a joint section 14b was cut out and used as a sample for Example 3. In addition, a joint section 14a of a disposable diaper produced using an anvil 92 without temporary fastening protrusions, with relatively high energy supplied to the ultrasonic horn during pressing of the front end section of each main fastening protrusion, was cut out and used as a sample for Example 2. Separately, joint sections 14a of two disposable diapers produced using an anvil 92 without temporary fastening protrusions but having main fastening protrusions with uniform heights from the outer peripheral surface of the anvil 92, and with the energy supplied to the ultrasonic horn being uniform among the main fastening protrusions, were each cut out and used as samples for Comparative Examples 1 and 2. The temporary fastening protrusions were circular shapes with diameters of 1 mm, and the main fastening protrusions were rounded rectangular shapes with short diameters of 1 mm and long diameters of 3 mm.

### (2) Width measurement

For each of the plurality of fused sections 21Db or plurality of fused sections 21Ub of the samples for Examples 1 to 3 and the Comparative Example produced in (1) above (joint section 14a or joint section 14b), an electron microscope was used to measure the widths of cross-sections of the perimeter wall section BW cut on a plane perpendicular to the longitudinal direction D1, for locations corresponding to the widths PI, P2 of the perimeter wall section BW of Fig. 4(a), and the widths of cross-sections of the perimeter wall section BW cut on a plane perpendicular to the transverse direction D2, for locations corresponding to widths P3a, P3b, P4a, P4b of the perimeter wall section BW of Fig. 4(a), and these were recorded as widths PI, P2, P3a, P3b, P4a, P4b for each location. A plane perpendicular to the prescribed direction refers to a plane crossing the prescribed direction at an angle of 90°±5°.

### (3) Peel-off test for joint section 14a or joint section 14b

By using a method of measuring the engaging strength of the joint sections 14a for the fused sections 21Db or fused sections 21Ub of the samples of the Examples and Comparative Examples produced in (1) above, the abdomen side section 11 and dorsal side section 13 were peeled apart and the peeled state was measured.
(i) Regions including the fused sections 21Db or fused sections 21Ub of each sample were cut to 25 mm widths and used as samples for an engaging strength test.
(ii) The ends in the lengthwise directions of the abdomen side section 11 and dorsal side section 13 of the sample for the engaging strength test were clamped with the chucks of a tensile tester (Model AG-lkNI, product of Shimadzu Corp.) (chuck distance: 10 mm).
(iii) The abdomen side section 11 and dorsal side section 13 of the sample for the engaging strength test were stretched with the tensile tester at a constant load (for example, 50 N) at 180° directions, so as to peel them apart.
(iv) A section corresponding to the fused sections 21Db or fused sections 21Ub of the sheet member of the abdomen side section 11 and the sheet member of the dorsal side section 13 that had been peeled apart was visually observed, and the presence or absence of tearing of the sheet member was confirmed. An evaluation of "O" (Pass) was assigned when the fused section where the sheet member had torn was not present, and an evaluation of "X" (Fail) was assigned when the fused section where the sheet member had torn was present, among the plurality of fused sections.

### (4) Measurement results

The measurement results are shown in Table 1 below.

P1/P2 (%) was 55, 69 and 78% for the samples of Examples 1 to 3, and 36% and 26% for the samples of Comparative Examples 1 and 2. That is, for the samples of Examples 1 to 3, the first dimension (P1) was equal to or more than 40% and less than 100% of the second dimension (P2), while the first dimension (P1) was less than 40% for the samples of Comparative Examples 1 and 2. The samples of Examples 1 to 3 were all "Pass", whereas the samples of Comparative Examples 1 and 2 were all "Fail". The results demonstrated that the first dimension is preferably equal to or more than 40% and less than 100% of the second dimension.

For the samples of Examples 1 to 3, the widths of the third perimeter wall section BW3 and fourth perimeter wall section BW4 were all increasing from the transverse direction first side D2I toward the transverse direction second side D2II, as indicated by P3a, P3b and P4a, P4b. This was also true for the samples of Comparative Examples 1 and 2.

P3a/P4a (%) was 98, 106 and 113% for the samples of Examples 1 to 3, and 107 and 106% for the samples of Comparative Examples 1 and 2. That is, for the samples of Examples 1 to 3, P3a was ±15% of P4a, i.e. they were approximately the same, as was also true for the samples of Comparative Examples 1 and 2.

For the samples of Examples 1 to 3, the first dimension P1 (50, 45, 83 µm) was 1.6, 1.5 and 2.8%, and therefore equal to or more than 0.5%, of the maximum dimension d2 of the core section BA in the transverse direction D2 (3 mm). For the samples of Comparative Examples 1 and 2, the first dimension P1 (59, 35 µm) was about 1.9 and 1.1% of the maximum dimension d2 (3 mm).

Furthermore, the first dimension P1 (50 µm) of the joint sections 14a of Example 1 was 42%, i.e. equal to or more than 40% and less than 100%, of the second dimension P2 (117 µm) of the joint sections 14b of Example 3.

When P3a and P4a were approximately equal to the minimum dimension of the third perimeter wall section BW3 or fourth perimeter wall section BW4, and P3b and P4b were approximately equal to the maximum dimension of the third perimeter wall section BW3 or fourth perimeter wall section BW4, the ratio of the minimum dimension and maximum dimension at the third perimeter wall section BW3 or fourth perimeter wall section BW4 (P3a/P3b, P4a/P4b) was 88, 90%; 94, 90%; 94, 82% for each of Examples 1 to 3, and therefore all were equal to or more than 60% and less than 100%. In Comparative Examples 1 and 2, on the other hand, they were 55, 50%; 55, 54%, and therefore both were less than 60%.

**[Table 1]**

| | Auxiliary fused section | P1 (µm) | P3a (µm) | P3b (µm) | P4a (µm) | P4b (µm) | P2 (µm) | P1/P2 (%) | Tearing | P3a/P4a (%) |
|---|---|---|---|---|---|---|---|---|---|---|
| Example 1 | Present (right) | 50 | 150 | 170 | 153 | 170 | 90 | 55 | ○ | 98 |
| Example 2 | Absent (right) | 45 | 120 | 128 | 113 | 126 | 65 | 69 | ○ | 106 |
| Example 3 | Present (left) | 130 | 175 | 187 | 155 | 190 | 166 | 78 | ○ | 113 |
| Comp. Example 1 | Absent (right) | 59 | 85 | 154 | 79 | 157 | 163 | 36 | X | 107 |
| Comp. Example 2 | Absent (right) | 35 | 85 | 121 | 79 | 145 | 133 | 26 | X | 106 |

### REFERENCE SIGNS LIST

1 Disposable diaper (absorbent article)
14a, 14b Joint section
21Db, 21Ub Fused section
11 Abdomen side section
13 Dorsal side section
BA Core section
BW Perimeter wall section
P1 First dimension
P2 Second dimension
D2I transverse direction (second direction) first side

## Claims

1. An absorbent article (1) having a longitudinal direction (D1), a transverse direction (D2) and a thickness direction (D3)that are mutually perpendicular, and comprising an abdomen side section (11) and a dorsal side section (13), both edges of the abdomen side section (11) and dorsal side section (13) in the transverse direction (D2) overlapping in the thickness direction (D3)along the longitudinal direction (D1) and being joined together by a pair of joint sections (14a, 14b), wherein
each of the pair of joint sections (14a, 14b) comprises a plurality of fused sections situated along the longitudinal direction (D1),
each of the plurality of fused sections includes:
a core section (BA) where a sheet member of the abdomen side section (11) and a sheet member of the dorsal side section (13) are fused together in the thickness direction, and
a perimeter wall section (BW) where the sheet member of the abdomen side section (11) and the sheet member of the dorsal side section (13) are fused together in the thickness direction, so as to surround the core section (BA) in a tubular manner in the thickness direction, and
when a direction in which a difference in widths between the perimeter wall section (BW) located on both outer sides of the core section (BA) is smaller is defined as a first direction and a direction in which the difference is larger is defined as a second direction, among the longitudinal direction (D1) and the transverse direction (D2),
in the second direction, a first dimension (P1) which is a width of the perimeter wall section (BW) located on a second direction first side that is one side of the core section (BA) in the second direction, is equal to or more than 40% and less than 100% of a second dimension (P2) which is a width of the perimeter wall section (BW) located on a second direction second side that is the other side of the core section (BA) in the second direction, wherein
in the first direction, widths of the perimeter wall section (BW) located on both outer sides of the core section (BA) both increase from the second direction first side toward the second direction second side.

2. The absorbent article according to claim 1, wherein
in the first direction, a third dimension, which is a width at an arbitrary location in the second direction of the perimeter wall section (BW) located on one outer side of the core section (BA), is ±15% of a fourth dimension, which is a width at a location opposite the arbitrary location of the perimeter wall section (BW) located on the other outer side of the core section (BA).

3. The absorbent article according to any one of claims 1 to 2, wherein
a maximum dimension of the core section (BA) in the second direction is longer than a maximum dimension of the core section (BA) in the first direction.

4. The absorbent article according to any one of claims 1 to 3, wherein
the first dimension (P1) of the perimeter wall section (BW) is equal to or more than 0.5% of a maximum dimension of the core section (BA) in the second direction.

5. The absorbent article according to any one of claims 1 to 4, wherein
the second direction is the transverse direction (D2), and
the first dimension (P1) of the perimeter wall section (BW) located on the second direction first side of the core section (BA) at a joint section on the second direction second side among the pair of joint sections (14a, 14b) is equal to or more than 40% and less than 100% of the second dimension (P2) of the perimeter wall section (BW) located on the second direction second side of the core section (BA) at a joint section on the second direction first side among the pair of joint sections (14a, 14b).

6. The absorbent article according to any one of claims 1 to 5, wherein
the second direction is the transverse direction (D2),
at least a joint section on the second direction second side, of the pair of joint sections (14a, 14b), further includes:
a plurality of auxiliary fused sections arranged along the longitudinal direction (D1) on the second direction first side of the plurality of fused sections,
each of the plurality of auxiliary fused sections includes:
an auxiliary core section (BA) where the sheet member of the abdomen side section (11) and the sheet member of the dorsal side section (13) are fused together in the thickness direction, and
an auxiliary perimeter wall section (BW) where the sheet member of the abdomen side section (11) and the sheet member of the dorsal side section (13) are fused together in the thickness direction, so as to surround the auxiliary core section (BA) in a tubular manner in the thickness direction, and
an area of the auxiliary core section (BA) of each of the plurality of auxiliary fused sections is smaller than an area of the core section (BA) of each of the plurality of fused sections, in the plan view.

7. The absorbent article according to claim 6, wherein
the pair of joint sections (14a, 14b) both further comprise the plurality of auxiliary fused sections on the second direction first side of the plurality of fused sections.

8. The absorbent article according to claim 6 or 7, wherein
a shape of the core section (BA) of each of the plurality of auxiliary fused sections are circular, in the plan view.

9. The absorbent article according to any one of claims 1 to 8, wherein
the second direction is the transverse direction (D2),
the absorbent article has a longitudinal center line running in the longitudinal direction (D1) through a center in the transverse direction (D2) of the absorbent article, and
the plurality of fused sections at the pair of joint sections (14a, 14b) are disposed along oblique directions with respect to the longitu-dinal direction (D1), gradually receding toward outer sides in the transverse direction (D2) from a waist side toward a leg side or from the leg side toward the waist side, of the absorbent article in the longitudinal direction (D1), with respect to the longitudinal center line.

10. A method of producing an absorbent article according to any one of claims 1 to 9, the method comprising:
a first forming step of, while transporting a continuous sheet member, in which semi-products of the absorbent article wherein the abdomen side section (11) and dorsal side section (13) are laminated together but not joined are continuously aligned in the transverse direction (D2), with the transverse direction (D2) as a machine direction, forming each of the plurality of fused sections in order with a time difference at locations corresponding to one of the pair of joint sections (14a, 14b) in the semi-product; and
a second forming step of forming each of the plurality of fused sections in order with a time difference at locations corresponding to the other of the pair of joint sections (14a, 14b) in the semi-product, after the first forming step,
wherein the each of the plurality of fused sections formed in the first and second forming steps includes:
a core section (BA) where a sheet member of the abdomen side section (11) and a sheet member of the dorsal side section (13) are fused together in the thickness direction (D3), and
a perimeter wall section (DW) where the sheet member of the abdomen side section (11) and the sheet member of the dorsal side section (13) are fused together in the thickness direction (D3), so as to surround the core section (BA) in a tubular manner in the thickness direction, and
when a direction in which a difference in widths between the perimeter wall section (BW) located on both outer sides of the core section (BA) is smaller is defined as a first direction and a direction in which the difference is larger is defined as a second direction, among the longitudinal direction (D1) and the transverse direction (D2),
in the second direction, a first dimension (P1) which is a width of the perimeter wall section (PW) located on a second direction first side that is one side of the core section (BA) in the second direction, is equal to or more than 40% and less than 100% of a second dimension (P2) which is a width of the perimeter wall section (BW) located on a second direction second side that is the other side of the core section (BA) in the second direction.

## Patentansprüche

1. Absorbierender Artikel (1) mit einer Längsrichtung (D1), einer Querrichtung (D2) und einer Dickenrichtung (D3), die zueinander senkrecht sind, und umfassend einen bauchseitigen Abschnitt (11) und einen rückenseitigen Abschnitt (13), wobei beide Ränder des bauchseitigen Abschnitts (11) und des rückenseitigen Abschnitts (13) in der Querrichtung (D2) in der Dickenrichtung (D3) entlang der Längsrichtung (D1) überlappen und durch ein Paar von Verbindungsabschnitten (14a, 14b) miteinander verbunden sind, wobei
jeder des Paars von Verbindungsabschnitten (14a, 14b) eine Mehrzahl von verschmolzenen Abschnitten umfasst, die entlang der Längsrichtung (D1) angeordnet sind,
jeder von der Mehrzahl von verschmolzenen Abschnitten umfasst:
einen Kernabschnitt (BA), in dem ein Lagenelement des bauchseitigen Abschnitts (11) und ein Lagenelement des rückenseitigen Abschnitts (13) in der Dickenrichtung miteinander verschmolzen sind, und
einen Umfangswandabschnitt (BW), in dem das Lagenelement des bauchseitigen Abschnitts (11) und das Lagenelement des rückenseitigen Abschnitts (13) in der Dickenrichtung miteinander so verschmolzen sind, dass sie den Kernabschnitt (BA) in der Dickenrichtung röhrenförmig umgeben, und
wenn, unter der Längsrichtung (D1) und der Querrichtung (D2), eine Richtung, in der ein Unterschied in den Breiten zwischen dem Umfangswandabschnitt (BW), der sich auf beiden Außenseiten des Kernabschnitts (BA) befindet, kleiner ist, wird sie als eine erste Richtung definiert, und eine Richtung, in der der Unterschied größer ist, wird als eine zweite Richtung definiert,
in der zweiten Richtung ist eine erste Abmessung (P1), die eine Breite des Umfangswandabschnitts (BW) ist, der sich auf einer ersten Seite der zweiten Richtung befindet, die eine Seite des Kernabschnitts (BA) in der zweiten Richtung ist, gleich oder mehr als 40% und weniger als 100% einer zweiten Abmessung (P2), die eine Breite des Umfangswandabschnitts (BW) ist, der sich auf einer zweiten Seite der zweiten Richtung befindet, die die andere Seite des Kernabschnitts (BA) in der zweiten Richtung ist, wobei
in der ersten Richtung die Breiten des Umfangswandabschnitts (BW), der sich auf beiden Außenseiten des Kernabschnitts (BA) befindet, beide von der ersten Seite der zweiten Richtung zur zweiten Seite der zweiten Richtung hin zunehmen.

2. Absorbierender Artikel nach Anspruch 1, wobei
in der ersten Richtung eine dritte Abmessung, die eine Breite an einer beliebigen Stelle in der zweiten Richtung des Umfangswandabschnitts (BW) ist, der sich auf einer Außenseite des Kernabschnitts (BA) befindet, ±15% einer vierten Abmessung ist, die eine Breite an einer Stelle gegenüber der beliebigen Stelle des Umfangswandabschnitts (BW) ist, der sich auf der anderen Außenseite des Kernabschnitts (BA) befindet.

3. Absorbierender Artikel nach einem der Ansprüche 1 bis 2, wobei
eine maximale Abmessung des Kernabschnitts (BA) in der zweiten Richtung länger ist als die maximale Abmessung des Kernabschnitts (BA) in der ersten Richtung.

4. Absorbierender Artikel nach einem der Ansprüche 1 bis 3, wobei
die erste Abmessung (P1) des Umfangswandabschnitts (BW) gleich oder größer als 0,5 % einer maximalen Abmessung des Kernabschnitts (BA) in der zweiten Richtung ist.

5. Absorbierender Artikel nach einem der Ansprüche 1 bis 4, wobei
die zweite Richtung die Querrichtung (D2) ist und
die erste Abmessung (P1) des Umfangswandabschnitts (BW), der sich auf der ersten Seite der zweiten Richtung des Kernabschnitts (BA) an einem Verbindungsabschnitt auf der zweiten Seite der zweiten Richtung von dem Paar von Verbindungsabschnitten (14a, 14b) befindet, gleich oder mehr als 40 % und weniger als 100 % der zweiten Abmessung (P2) des Umfangswandabschnitts (BW) beträgt, der sich auf der zweiten Seite der zweiten Richtung des Kernabschnitts (BA) an einem Verbindungsabschnitt auf der ersten Seite der zweiten Richtung von dem Paar von Verbindungsabschnitten (14a, 14b) befindet.

6. Absorbierender Artikel nach einem der Ansprüche 1 bis 5, wobei
die zweite Richtung die Querrichtung (D2) ist,
mindestens ein Verbindungsabschnitt auf der zweiten Seite der zweiten Richtung des Paares von Verbindungsabschnitten (14a, 14b) ferner umfasst:
eine Mehrzahl von zusätzlichen verschmolzenen Abschnitten, die entlang der Längsrichtung (D1) auf der ersten Seite der zweiten Richtung der Mehrzahl von verschmolzenen Abschnitten angeordnet sind,
jeder von der Mehrzahl von zusätzlichen verschmolzenen Abschnitten umfasst:
einen zusätzlichen Kernabschnitt (BA), in dem das Lagenelement des bauchseitigen Abschnitts (11) und das Lagenelement des rückenseitigen Abschnitts (13) in der Dickenrichtung miteinander verschmolzen sind, und
einen zusätzlichen Umfangswandabschnitt (BW), in dem das Lagenelement des bauchseitigen Abschnitts (11) und das Lagenelement des rückenseitigen Abschnitts (13) in der Dickenrichtung miteinander so verschmolzen sind, dass sie den zusätzlichen Kernabschnitt (BA) in der Dickenrichtung röhrenförmig zu umgeben, und
in der Draufsicht eine Fläche des zusätzlichen Kernabschnitts (BA) von jedem der Mehrzahl von verschmolzenen zusätzlichen Abschnitte kleiner ist als eine Fläche des Kernabschnitts (BA) von jedem der Mehrzahl verschmolzenen Abschnitte.

7. Absorbierender Artikel nach Anspruch 6, wobei
das Paar von Verbindungsabschnitten (14a, 14b) beide ferner die Mehrzahl von zusätzlichen verschmolzenen Abschnitten auf der ersten Seite der zweiten Richtung der Mehrzahl von verschmolzenen Abschnitten umfassen.

8. Absorbierender Artikel nach Anspruch 6 oder 7, wobei
eine Form des Kernabschnitts (BA) von jedem der Mehrzahl von verschmolzenen Hilfsabschnitten in der Draufsicht kreisförmig ist.

9. Absorbierender Artikel nach einem der Ansprüche 1 bis 8, wobei
die zweite Richtung die Querrichtung (D2) ist,
der absorbierende Artikel eine Längsmittellinie aufweist, die in der Längsrichtung (D1) durch einen Mittelpunkt in der Querrichtung (D2) des absorbierenden Artikels verläuft, und
die Mehrzahl von verschmolzenen Abschnitten an dem Paar von Verbindungsabschnitten (14a, 14b) entlang von in Bezug auf die Längsrichtung (D1) schrägen Richtungen angeordnet sind und allmählich in Richtung zu Außenseiten in der Querrichtung (D2) von einer Taillenseite zu einer Beinseite oder von der Beinseite zu der Taillenseite des absorbierenden Artikels in der Längsrichtung (D1) in Bezug auf die Längsmittellinie zurückweichen.

10. Verfahren zum Herstellen eines absorbierenden Artikels nach einem der Ansprüche 1 bis 9, wobei das Verfahren umfasst:
einen ersten Formungsschritt, bei dem während des Transports eines durchgehenden Lagenelements, bei dem Halbprodukte des absorbierenden Artikels, bei denen der bauchseitige Abschnitt (11) und der rückenseitige Abschnitt (13) zusammenlaminiert, aber nicht verbunden sind, kontinuierlich in der Querrichtung (D2) ausgerichtet werden, wobei die Querrichtung (D2) eine Maschinenrichtung ist, wobei jeder von der Mehrzahl von verschmolzenen Abschnitten in einer Reihenfolge mit einer Zeitdifferenz an Stellen geformt wird, die einem von dem Paar von Verbindungsabschnitten (14a, 14b) in dem Halbprodukt entsprechen; und
einen zweiten Formungsschritt des Formens von jedem von der Mehrzahl von verschmolzenen Abschnitten in Reihenfolge mit einer Zeitdifferenz an Stellen, die dem anderen von dem Paar von Verbindungsabschnitten (14a, 14b) in dem Halbprodukt entsprechen, nach dem ersten Formungsschritt,
wobei jeder von der Mehrzahl von in den ersten und zweiten Formungsschritten geformten verschmolzenen Abschnitten umfasst:
einen Kernabschnitt (BA), in dem ein Lagenelement des bauchseitigen Abschnitts (11) und ein Lagenelement des rückenseitigen Abschnitts (13) in der Dickenrichtung miteinander verschmolzen sind, und
einen Umfangswandabschnitt (BW), in dem das Lagenelement des bauchseitigen Abschnitts (11) und das Lagenelement des rückenseitigen Abschnitts (13) in der Dickenrichtung miteinander so verschmolzen sind, dass sie den Kernabschnitt (BA) in der Dickenrichtung röhrenförmig umgeben, und
wenn eine Richtung, in der ein Unterschied in den Breiten zwischen dem Umfangswandabschnitt (BW), der sich auf beiden Außenseiten des Kernabschnitts (BA) befindet, kleiner ist, wird sie als eine erste Richtung definiert, und eine Richtung, in der der Unterschied größer ist, wird als eine zweite Richtung definiert, unter der Längsrichtung (D1) und der Querrichtung (D2),
in der zweiten Richtung ist eine erste Abmessung (P1), die eine Breite des Umfangswandabschnitts (BW) ist, der sich auf einer ersten Seite der zweiten Richtung befindet, die eine Seite des Kernabschnitts (BA) in der zweiten Richtung ist, gleich oder mehr als 40% und weniger als 100% einer zweiten Abmessung (P2), die eine Breite des Umfangswandabschnitts (BW) ist, der sich auf einer zweiten Seite der zweiten Richtung befindet, die die andere Seite des Kernabschnitts (BA) in der zweiten Richtung ist.

## Revendications

1. Article absorbant (1) présentant un sens longitudinal (D1), un sens transversal (D2) et un sens de l'épaisseur (D3) qui sont mutuellement perpendiculaires, et comprenant une section côté abdomen (11) et une section côté dorsal (13), les deux bords de la section côté abdomen (11) et de la section côté dorsal (13) dans le sens transversal (D2) se chevauchant dans le sens de l'épaisseur (D3) le long du sens longitudinal (D1) et étant assemblés ensemble par une paire de sections d'assemblage (14a, 14b), dans lequel
chacune de la paire de sections d'assemblage (14a, 14b) comprend une pluralité de sections thermocollées situées le long du sens longitudinal (D1),
chacune de la pluralité de sections thermocollées comporte :
une section centrale (BA) où un élément en feuille de la section côté abdomen (11) et un élément en feuille de la section côté dorsal (13) sont thermocollés dans le sens de l'épaisseur, et
une section de paroi périmétrique (BW) où l'élément en feuille de la section côté abdomen (11) et l'élément en feuille de la section côté dorsal (13) sont thermocollés ensemble dans le sens de l'épaisseur, de manière à entourer la section centrale (BA) de manière tubulaire dans le sens de l'épaisseur, et
lorsqu'un sens dans lequel une différence de largeur entre la section de paroi périmétrique (BW) située sur les deux côtés extérieurs de la section centrale (BA) est plus petite est défini comme un premier sens et un sens dans lequel la différence est plus grande est défini comme un second sens, parmi le sens longitudinal(D1) et le sens transversal (D2),
dans le second sens, une première dimension (P1) qui est une largeur de la section de paroi périmétrique (BW) située sur un premier côté du second sens qui est un côté de la section centrale (BA) dans le second sens, est égale ou supérieure à 40% et à moins de 100% d'une deuxième dimension (P2) qui est une largeur de la section de paroi périmétrique (BW) située sur un second côté du second sens qui est l'autre côté de la section centrale (BA) dans le second sens, dans lequel
dans le premier sens, les largeurs de la section de paroi périmétrique (BW) situées des deux côtés extérieurs de la section centrale (BA) augmentent du premier côté du second sens vers le second côté du second sens.

2. Article absorbant selon la revendication 1, dans lequel
dans le premier sens, une troisième dimension, qui est une largeur à un emplacement arbitraire dans le second sens de la section de paroi périmétrique (BW) située sur un côté extérieur de la section centrale (BA), représente ±15% d'une quatrième dimension, qui est une largeur à un emplacement opposé à l'emplacement arbitraire de la section de paroi périmétrique (BW) située sur l'autre côté extérieur de la section centrale (BA).

3. Article absorbant selon l'une quelconque des revendications 1 à 2, dans lequel
une dimension maximale de la section centrale (BA) dans le second sens est plus longue qu'une dimension maximale de la section centrale (BA) dans le premier sens.

4. Article absorbant selon l'une quelconque des revendications 1 à 3, dans lequel :
la première dimension (P1) de la section de paroi périmétrique (BW) est égale ou supérieure à 0,5 % d'une dimension maximale de la section centrale (BA) dans le second sens.

5. Article absorbant l'une quelconque des revendications 1 à 4, dans lequel
le second sens est le sens transversal (D2), et
la première dimension (P1) de la section de paroi périmétrique (BW) située sur le premier côté du second sens de la section centrale (BA), au niveau d'une section d'assemblage sur le second côté du second sens parmi la paire de sections d'assemblage (14a, 14b) est égale ou supérieure à 40 % et inférieure à 100 % de la deuxième dimension (P2) de la section de paroi périmétrique (BW) située sur le second côté du second sens de la section centrale (BA), au niveau d'une section d'assemblage, sur le premier côté du second sens parmi la paire de sections d'assemblage (14a, 14b).

6. Article absorbant selon l'une quelconque des revendications 1 à 5, dans lequel
le second sens est le sens transversal (D2),
au moins une section d'assemblage sur le second côté du second sens, de la paire de sections d'assemblage (14a, 14b), comporte en outre :
une pluralité de sections auxiliaires thermocollées agencées le long du sens longitudinal (D1) sur le premier côté du second sens de la pluralité de sections thermocollées,
chacune de la pluralité de sections auxiliaires thermocollées comporte :
une section centrale auxiliaire (BA) où l'élément en feuille de la section côté abdomen (11) et l'élément en feuille de la section côté dorsal (13) sont thermocollés ensemble dans le sens de l'épaisseur, et
une section de paroi périmétrique auxiliaire (BW) où l'élément en feuille de la section côté abdomen (11) et l'élément en feuille de la section côté dorsal (13) sont thermocollés ensemble dans le sens de l'épaisseur, de manière à entourer la section centrale auxiliaire (BA) de manière tubulaire dans le sens de l'épaisseur, et
une zone de la section centrale auxiliaire (BA) de chacune de la pluralité de sections auxiliaires thermocollées est plus petite qu'une zone de la section centrale (BA) de chacune de la pluralité de sections thermocollées, dans la vue en plan.

7. Article absorbant selon la revendication 6, dans lequel
la paire de sections d'assemblage (14a, 14b) comprend en outre la pluralité de sections auxiliaires thermocollées sur le premier côté du second sens de la pluralité de sections thermocollées.

8. Article absorbant selon la revendication 6 ou 7, dans lequel
une forme de la section centrale (BA) de chacune de la pluralité de sections auxiliaires thermocollées est circulaire, dans la vue en plan.

9. Article absorbant selon l'une quelconque des revendications 1 à 8, dans lequel
le second sens est le sens transversal (D2),
l'article absorbant a un axe longitudinal dans le sens longitudinal (D1) à travers un centre dans le sens transversal (D2) de l'article absorbant, et
la pluralité des sections thermocollées au niveau de la paire de sections d'assemblage (14a, 14b) est agencée suivant des directions obliques par rapport au sens longitudinal (D1), en s'éloignant progressivement vers des côtés extérieurs dans le sens transversal (D2) d'un côté taille vers un côté jambe ou du côté jambe vers le côté taille, de l'article absorbant dans le sens longitudinal (D1), par rapport à l'axe longitudinal.

10. Procédé de production d'un article absorbant selon l'une quelconque des revendications 1 à 9, le procédé comprenant :
une première étape de formation, lors du transport d'un élément en feuille continu, dans lequel des produits semi-finis de l'article absorbant dans lequel la section côté abdomen (11) et la section côté dorsal (13) sont stratifiées ensemble mais non assemblées sont alignés en continu dans le sens transversal (D2), le sens transversal (D2) étant un sens de la machine, formant chacune de la pluralité de sections thermocollées dans l'ordre avec une différence de temps à des emplacements correspondant à une section de la paire de sections d'assemblage (14a, 14b) dans le produit semi-fini ; et
une seconde étape de formation de la formation de chacune de la pluralité de sections thermocollées dans l'ordre avec une différence de temps à des emplacements correspondant à l'autre de la paire de sections d'assemblage (14a, 14b) dans le produit semi-fini, après la première étape de formation,
dans lequel chacune de la pluralité de sections thermocollées formée aux première et seconde étapes de formation comporte :
une section centrale (BA) où un élément en feuille de la section côté abdomen (11) et un élément en feuille de la section côté dorsal (13) sont thermocollés ensemble dans le sens de l'épaisseur (D3), et
une section de paroi périmétrique (DW) où l'élément en feuille de la section côté abdomen (11) et l'élément en feuille de la section côté dorsal (13) sont thermocollés ensemble dans le sens de l'épaisseur (D3), de manière à entourer la section centrale (BA) de manière tubulaire dans le sens de l'épaisseur, et
lorsqu'un sens dans lequel une différence de largeur entre la section de paroi périmétrique (BW) située sur les deux côtés extérieurs de la section centrale (BA) est plus petite est défini comme un premier sens et un sens dans lequel la différence est plus grande est défini comme un second sens, parmi le sens longitudinal (D1) et le sens transversal (D2),
dans le second sens, une première dimension (P1) qui est une largeur de la section de paroi périmétrique (PW) située sur un premier côté du second sens qui est un côté de la section centrale (BA) dans le second sens, est égale ou supérieure à 40 % et inférieure à 100 % d'une deuxième dimension (P2) qui est une largeur de la section de paroi périmétrique (BW) située sur un second côté du second sens qui est l'autre côté de la section centrale (BA) dans le second sens.
